# EUROPÄISCHE PATENTSCHRIFT

(11) **EP 1 292 271 B1**
(45) Veröffentlichungstag und Bekanntmachung des Hinweises auf die Patenterteilung: **08.11.2006**
(21) Anmeldenummer: 01957836.8
(22) Anmeldetag: 09.06.2001
(51) Int. Cl.: A61Q 5/10, A61K 8/49

(54) **MITTEL ZUM FÄRBEN VON KERATINHALTIGEN FASERN**
AGENT FOR DYEING FIBRES CONTAINING KERATIN
AGENT POUR TEINDRE DES FIBRES CONTENANT DE LA KERATINE

(30) Priorität: 17.06.2000 DE 10029929
(43) Veröffentlichungstag der Anmeldung: 19.03.2003
(73) Patentinhaber: Henkel Kommanditgesellschaft auf Aktien, 40589 Düsseldorf (DE)
(72) Erfinder: OBERKOBUSCH, Doris, 40591 Düsseldorf (DE); HÖFFKES, Horst, 40595 Düsseldorf (DE); MÖLLER, Hinrich, 40789 Monheim (DE); GROSS, Wibke, 40549 Düsseldorf (DE); MARTIN, Hans-Dieter, 40591 Düsseldorf (DE)
(86) Internationale Anmeldenummer: PCT/EP2001/006544
(87) Internationale Veröffentlichungsnummer: WO 2001/097754

(56) Entgegenhaltungen:
- EP-A- 0 984 010
- WO-A-99/51688
- DE-A- 4 206 537
- DE-A- 19 937 289
- GB-A- 1 436 589
- US-A- 3 376 284
- US-A- 5 089 025

## Beschreibung

Die Erfindung betrifft ein Mittel zum Färben von keratinhaltigen Fasern, insbesondere menschlichen Haaren, das mindestens ein Chinoxalinderivat enthält, die Verwendung dieser Verbindungen in Mitteln zum Färben von keratinhaltigen Fasern sowie ein Verfahren zum Färben von keratinhaltigen Fasern, insbesondere menschlichen Haaren.

Für das Färben von keratinhaltigen Fasern, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, p-Phenylendiamin, 2,4-Diaminophenoxyethanol, 2-Amino-4-(2-hydroxyethylamino)-anisol (Lehmanns Blau), 1-Phenyl-3-methyl-pyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsunternehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch i.a. unter dem Einfluss von Oxidationsmitteln wie z. B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, dass die Färbungen häufig nur über unzureichende Echtheitseigenschaften verfügen.

Die Verwendung der unten näher beschriebenen Chinoxalinderivate zum Färben von keratinhaltigen Fasern ist bislang nicht bekannt.

Aufgabe der vorliegenden Erfindung ist es, Färbemittel für Keratinfasern, insbesondere menschliche Haare, bereitzustellen, die hinsichtlich der Farbtiefe, der Grauabdeckung und den Echtheitseigenschaften qualitativ den üblichen Oxidationshaarfärbemitteln mindestens gleichwertig sind, ohne jedoch unbedingt auf Oxidationsmittel wie z. B. H₂O₂ angewiesen zu sein. Darüber hinaus dürfen die Färbemittel kein oder lediglich ein sehr geringes Sensibilisierungspotential aufweisen.

Überraschenderweise wurde nun gefunden, dass die in der Formel I dargestellten Chinoxalinderivate sich auch in Abwesenheit von oxidierenden Agentien hervorragend zum Färben von keratinhaltigen Fasern eignen. Sie ergeben Ausfärbungen mit hervorragender Brillanz und Farbtiefe und führen zu vielfältigen Farbnuancen. Der Einsatz von oxidierenden Agentien soll dabei jedoch nicht prinzipiell ausgeschlossen werden.

Gegenstand der Erfindung ist ein Mittel zum Färben von keratinhaltigen Fasern, insbesondere menschlichen Haaren, enthaltend mindestens ein Chinoxalinderivat mit der Formel la oder Ib oder Ic 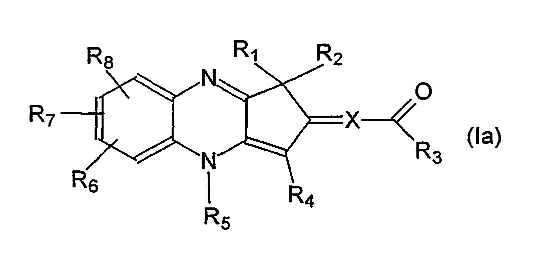 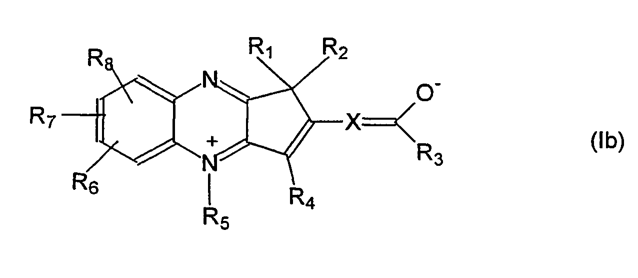 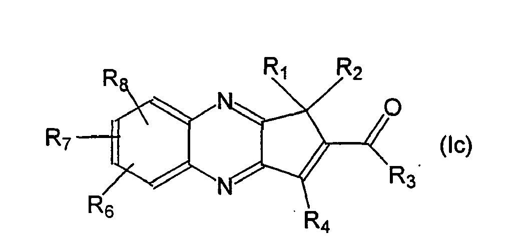 in der bedeuten
R¹, R², R³, R⁴ unabhängig voneinander ein Wasserstoffatom oder eine C₁-C₄₋Alkylgruppe,
R⁵ ein Wasserstoffatom, eine C₁-C₆-Alkyl- oder Aralkyl-, Aryl-, C₁-C₄-Alkenyl-, Hydroxyalkyl-, Carboxyalkylgruppe,
R⁶, R⁷, R⁸ unabhängig voneinander ein Wasserstoff-, Halogenatom, eine C₁-C₄-Alkyl-, C₁₋C₄-Alkoxy-, Nitro-, Amino-, Alkylamino-, Hydroxy-, Carboxy-, Sulfo-, Thiol-, oder C₁-C₄₋Alkylthiogruppe, wobei je zwei Reste auch gemeinsam einen Ring bilden können,
wobei -X= bzw. =X- für -CH=, =CH- steht.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie z. B. Regeneratcellulose, Nitro-, Alkyl- oder Hydroxyalkyl- oder Acetylcellulose und synthetischer Fasern, wie z. B. Polyamid-, Polyacrylnitril-, Polyurethan- und Polyesterfasern verwendet werden.

Die Verbindungen mit den Formeln Ia oder Ib oder Ic sind zum großen Teil literaturbekannt; sie sind im Handel erhältlich oder nach bekannten Syntheseverfahren herstellbar.

Vorzugsweise sind die Verbindungen mit der Formel la oder Ib oder Ic ausgewählt aus 1,1,3-Trimethyl-1H-cyclopenta[b]chinoxalin-2-carbaldehyd, 6(7)-Chlor-, 6(7)-Fluor-, 6(7)-Brom-, 6(7)-Methyl-, 6(7)-Nitro-, 6,7-Dichlor-, 6,7-Methylendioxy-1,1,3-trimethyl-1H-cyclopenta[b]chinoxalin-2-carbaldehyd, 1,1,3,6(7)-Tetramethyl-, 1,1,3,6,7-Pentamethyl-1H-cyclopenta[b]chinoxalin-2-carbaldehyd, 1,1,3-Trimethyl-1H-benzo[g]cyclopenta[b]chinoxalin-2-carbaldehyd, 2,4-Dihydro-1,1,3,4-tetramethyl-1H-cyclopenta[b]chinoxalin-2-yliden-acetaldehyd, 2,4-Dihydro-4-phenyl-1,1,3-trimethyl-1H-cyclopenta[b]chinoxalin-2-yliden-acetaldehyd, 6,7-Dichlor-2,4-dihydro-1,1,3,4-tetramethyl-1H-cyclopenta[b]chinoxalin-2-yliden-acetaldehyd, 7-Chlor-2,4-dihydro-4-(2-propyl)-1,1,3-trimethyl-1H-cyclopenta[b]chinoxalin-2-yliden-acetaldehyd, 2,4-Dihydro-6,7-methylendioxy-1,1,3,4-tetramethyl-1 H-cyclopenta[b]chinoxalin-2-yliden-acetaldehyd sowie deren beliebigen Gemischen.

Die voranstehend genannten Chinoxalinderivate mit der Formel la oderlb oder Ic werden vorzugsweise in den erfindungsgemäßen Mitteln in einer Menge von 0,03 bis 65 mmol, insbesondere von 1 bis 40 mmol, bezogen auf 100 g des gesamten Färbemittels, verwendet. Sie können als direktziehende Färbemittel oder in Gegenwart von üblichen Oxidationsfarbstoffvorprodukten eingesetzt werden.

Färbemittel, die als färbende Komponente Chinoxalinderivate mit der Formel la oder Ib oder Ic allein enthalten, werden bevorzugt für hellere Färbungen eingesetzt. Färbungen mit noch erhöhter Brillanz und verbesserten Echtheitseigenschaften (Lichtechtheit, Waschechtheit, Reibechtheit) über einen weiten Nuancenbereich von gelb über purpur, braun bis hin zu tiefblau werden erzielt, wenn die erfindungsgemäß eingesetzten Verbindungen mit der Formel Ia oder Ib oder Ic gemeinsam mit mindestens einer weiteren Komponente (im folgenden Komponente B genannt), ausgewählt aus Verbindungen mit primärer oder sekundärer Aminogruppe oder Hydroxygruppe, ausgewählt aus primären oder sekundären aromatischen Aminen, stickstoffhaltigen heterocyclischen Verbindungen und aromatischen Hydroxyverbindungen, Aminosäuren; aus 2 bis 9 Aminosäuren aufgebauten Oligopeptiden und/oder CH-aktiven Verbindungen, verwendet werden. Dies sind einerseits Verbindungen, die für sich alleine keratinhaltige Fasern nur schwach färben und erst gemeinsam mit den Verbindungen der Formel Ia oder Ib oder Ic brillante Färbungen ergeben. Andererseits sind darunter aber auch Verbindungen, die bereits als Oxidationsfarbstoffvorprodukte eingesetzt werden.

In allen Färbemitteln können auch mehrere verschiedene Chinoxalinderivate der Formel Ia oder Ib oder Ic gemeinsam zum Einsatz kommen; ebenso können auch mehrere verschiedene Verbindungen der Komponente B gemeinsam verwendet werden.

Unter die voranstehend beschriebene Ausführungsform fällt auch die Verwendung von solchen Substanzen, die Reaktionsprodukte von erfindungsgemäß eingesetzten Chinoxalinderivaten mit den genannten Verbindungen der Komponente B darstellen, als direktziehende Färbemittel. Derartige Reaktionsprodukte können z. B. durch kurzes Erwärmen der beiden Komponenten in stöchiometrischen Mengen in wässrigem neutralen bis schwach alkalischen Milieu erhalten werden, wobei sie entweder als Feststoff aus der Lösung ausfallen oder durch Eindampfen der Lösung daraus isoliert werden. Die Reaktionsprodukte können auch in Kombination mit anderen Farbstoffen oder Farbstoffvorprodukten eingesetzt werden.

Geeignete Verbindungen mit primärer oder sekundärer Aminogruppe als Komponente B sind z. B. primäre aromatische Amine wie N,N-Dimethyl-, N,N-Diethyl-, N-(2-Hydroxyethyl)-N-ethyl-, N,N-Bis-(2-hydroxyethyl)-, N-(2-Methoxyethyl-), 2,3-, 2,4-, 2,5-Dichlor-p-phenylendiamin, 2-Chlor-p-phenylendiamin, 2,5-Dihydroxy-4-morpholinoanilin-dihydrobromid, 2-, 3-, 4-Aminophenol, 2-Aminomethyl-4-aminophenol, 2-Hydroxymethyl-4-aminophenol, o-, p-Phenylendiamin, o-Toluylendiamin, 2,5-Diaminotoluol, -phenol, - phenethol, 4-Amino-3-methylphenol, 2-(2,5-Diaminophenyl)-ethanol, 2,4-Diaminophenoxyethanol, 2-(2,5-Diaminophenoxy)-ethanol, 4-Methylamino-, 3-Amino-4-(2'-hydroxyethyloxy)-, 3,4-Methylendiamino-, 3,4-Methylendioxyanilin, 3-Amino-2,4-dichlor-, 4-Methylamino-, 2-Methyl-5-amino-, 3-Methyl-4-amino-, 2-Methyl-5-(2-hydroxyethylamino)-, 6-Methyl-3-amino-2-chlor-, 2-Methyl-5-amino-4-chlor-, 3,4-Methylendioxy-, 5-(2-Hydroxyethylamino)-4-methoxy-2-methyl-, 4-Amino-2-hydroxymethylphenol, 1,3-Diamino-2,4-dimethoxybenzol, 2-, 3-, 4-Aminobenzoesäure, -phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxy-benzoesäure, 2-, 3-, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 3,5-Diamino-4-hydroxybrenzcatechin, 1,4-Bis-(4-aminophenyl)-1,4-diazacycloheptan, Bis-(5-amino-2-hydroxyphenyl)-methan, aromatische Nitrile, Nitrogruppen-haltige Aminoverbindungen, wie 3-Amino-6-methylamino-2-nitro-pyridin, Pikraminsäure, 1-(2(3)-Nitro-4-amino)-phenylazo-2-hydroxy-7-trimethylammoniumnaphthalin-chlorid, 1-Hydroxy-2-amino-4,6-dinitro-benzol, 1-Amino-2-nitro-4-bis-(2-hydroxyethyl)-amino-benzol, 1-Amino-2-(2-hydroxyethyl)-amino-5-nitrobenzol (HC Yellow Nr. 5), 1-Amino-2-nitro-4-(2-hydroxyethyl)-aminobenzol (HC Red Nr. 7), 2-Chloro-5-nitro-N-hydroxyethyl-1,4-phenylendiamin, 1-(2-Hydroxyethyl)-amino-2-nitro-4-amino-benzol (HC Red Nr. 3), 4-Amino-3-nitrophenol, 4-Amino-2-nitrophenol, 6-Nitro-o-toluidin, 1-Amino-3-methyl-4-(2-hydroxyethyl)-amino-6-nitrobenzol (HC Violet Nr. 1), 1-Amino-2-nitro-4-(2,3-dihydroxypropyl)-amino-5-chlorobenzol (HC Red Nr. 10), 4-Amino-2-nitrodiphenyl-amino-2'-carbonsäure (2-(4-Amino-2-nitroanilino)-benzoesäure), 6-Nitro-2,5-diaminopyridin, 2-Amino-6-chloro-4-nitrophenol, 2-Amino-6-chloro-4-nitrophenol, 1-Amino-2-(3-nitrophenylazo)-7-phenylazo-8-naphthol-3,6-disulfonsäure, Dinatriumsalz (Acid blue Nr. 29), 1-Amino-2-(2-hydroxy-4-nitrophenylazo)-8-naphthol-3,6-disulfonsäure, Dinatriumsalz (Palatinchrome green), 1-Amino-2-(3-chlor-2-hydroxy-5-nitrophenylazo)-8-naphthol-3,6-disulfonsäure, Dinatriumsalz (Gallion), 4-Amino-4'-nitrostilben-2,2'-disulfonsäure, Dinatriumsalz, 2,4-Diamino-3',5'-dinitro-2'-hydroxy-5-methyl-azo-benzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2-nitrobenzylidenamino)-benzol, 2-[2-(Diethylamino)-ethylamino]-5-nitroanilin, 3-Amino-4-hydroxy-5-nitrobenzolsulfonsäure, 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 2-Amino-6-chlor-4-nitrophenol, 1-Amino-5-chlor-4-(2-hydroyethylamino)-2-nitrobenzol, aromatische Aniline bzw. Phenole mit einem weiteren aromatischen Rest, wie sie in der Formel II dargestellt sind 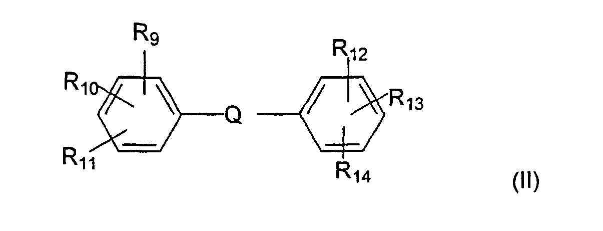 in der R⁹ für eine Hydroxy- oder eine Aminogruppe, die durch C₁₋₄-Alkyl-, C₁₋₄-Hydroxyalkyl-, C₁₋₄-Alkoxy- oder C₁₋₄-Alkoxy-C₁₋₄-alkylgruppen substituiert sein kann, steht,
R¹⁰, R¹¹, R¹², R¹³ und R¹⁴ für Wasserstoff, eine Hydroxy- oder eine Aminogruppe, die durch C₁₋₄-Alkyl-, C₁₋₄-Hydroxyalkyl, C₁₋₄-Alkoxy-, C₁₋₄-Aminoalkyl- oder C₁₋₄-Alkoxy-C₁₋₄₋alkylgruppen substituiert sein kann, stehen, und
Q für eine direkte Bindung, eine gesättigte oder ungesättigte, ggf. durchHydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel III

Z-(CH₂-Y-CH₂-Z')ₒ (III)

in der Y eine direkte Bindung, eine CH₂- oder CHOH-Gruppe bedeutet,
Z und Z' unabhängig voneinander für ein Sauerstoffatom, eine NR¹⁵-Gruppe, worin R¹⁵ Wasserstoff, eine C₁₋₄-Alkyl- oder eine Hydroxy-C₁₋₄-alkylgruppe bedeutet, die Gruppe O-(CH₂)ₚ-NH oder NH-(CH₂)ₚ'-O, worin p und p' 2 oder 3 sind, stehen und
o eine Zahl von 1 bis 4 bedeutet,
wie beispielsweise 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorid, 4,4'-Diaminodiphenylmethan, -sulfid, -sulfoxid, -amin, 4,4'-Diaminodiphenylamin-2-sulfonsäure, 4,4'-Diaminobenzophenon, -diphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, -2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin.

Die vorgenannten Verbindungen können sowohl in freier Form als auch in Form ihrer physiologisch verträglichen Salze, insbesondere als Salze anorganischer Säuren, wie Salz- oder Schwefelsäure, eingesetzt werden.

Geeignete stickstoffhaltige heterocyclische Verbindungen sind z. B. 2-, 3-, 4-Amino-, 2-Amino-3-hydroxy-, 2,6-Diamino-, 2,5-Diamino-, 2,3-Diamino-, 2-Dimethylamino-5-amino-, 2-Methylamino-3-amino-6-methoxy-, 2,3-Diamino-6-methoxy-, 2,6-Dimethoxy-3,5-diamino-, 2,4,5-Triamino-, 2,6-Dihydroxy-3,4-dimethylpyridin, 2,4-Dihydroxy-5,6-diamino-, 4,5,6-Triamino-, 4-Hydroxy-2,5,6-triamino-, 2-Hydroxy-4,5,6-triamino-, 2,4,5,6-Tetraamino-, 2-Methylamino-4,5,6-triamino-, 2,4-, 4,5-Diamino-, 2-Amino-4-methoxy-6-methyl-pyrimidin, 3,5-Diaminopyrazol, -1,2,4-triazol, 3-Amino-, 3-Amino-5-hydroxypyrazol, 4,5-Diamino-1-(2-hydroxyethyl)-pyrazol, 2-, 3-, 8-Aminochinolin, 4-Aminochinaldin, 2-, 6-Aminonicotinsäure, 5-Aminoisochinolin, 5-, 6-Aminoindazol, 5-, 7-Aminobenzimidazol, -benzothiazol, 2,5-Dihydroxy-4-morpholinoanilin sowie Indol- und Indolinderivaten, wie 4-, 5-, 6-, 7-Aminoindol, 5,6-Dihydroxyindol, 5,6-Dihydroxyindolin und 4-Hydroxyindolin. Weiterhin als heterocyclische Verbindungen können erfindungsgemäß die in der DE-U1-299 08 573 offenbarten Hydroxypyrimidine eingesetzt werden. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Geeignete aromatische Hydroxyverbindungen sind z. B. 2-, 4-, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2-Hydroxyethyl)-, 3,4-Methylendioxyphenol, 2,4-, 3,4-Dihydroxybenzoesäure, -phenylessigsäure, Gallussäure, 2,4,6-Trihydroxybenzoesäure, -acetophenon, 2-, 4-Chlorresorcin, 1-Naphthol, 1,5-, 2,3-, 2,7-Dihydroxynaphthalin, 6-Dimethylamino-4-hydroxy-2-naphthalinsulfonsäure, 3,6-Dihydroxy-2,7-naphthalinsulfonsäure.

Als Aminosäuren kommen bevorzugt alle natürlich vorkommenden und synthetischen α-Aminosäuren in Frage, z.B. die durch Hydrolyse aus pflanzlichen oder tierischen Proteinen, z.B. Kollagen, Keratin, Casein, Elastin, Sojaprotein, Weizengluten oder Mandelprotein zugänglichen Aminosäuren. Dabei können sowohl sauer als auch alkalisch reagierende Aminosäuren eingesetzt werden. Bevorzugte Aminosäuren sind Arginin, Histidin, Tyrosin, Phenylalanin, DOPA (Dihydroxyphenylalanin), Ornithin, Lysin und Tryptophan. Aber auch andere Aminosäuren, wie z.B. 6-Aminocapronsäure, können eingesetzt werden.

Die Oligopeptide können dabei natürlich vorkommende oder synthetische Oligopeptide, aber auch die in Polypeptid- oder Proteinhydrolysaten enthaltenen Oligopeptide sein, sofern sie über eine für die Anwendung in den erfindungsgemäßen Färbemitteln ausreichende Wasserlöslichkeit verfügen. Als Beispiele sind z.B. Glutathion oder die in den Hydrolysaten von Kollagen, Keratin, Casein, Elastin, Sojaprotein, Weizengluten oder Mandelprotein enthaltenen Oligopeptide zu nennen. Bevorzugt ist dabei die Verwendung gemeinsam mit Verbindungen mit primärer oder sekundärer Aminogruppe oder mit aromatischen Hydroxyverbindungen.

Als CH-aktive Verbindungen können beispielhaft genannt werden 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, Fischersche Base (1,3,3-Trimethyl-2-methylenindolin), 2,3-Dimethyl-benzothiazoliumiodid, 2,3-Dimethyl-benzothiazolium-p-toluolsulfonat, 1,2-Dimethyl-naphtho[1,2-d]thiazolium-p-toluolsulfonat, 1-Ethyl-2-methyl-naphtho[1,2-d]thiazolium-p-toluolsulfonat, Rhodanin, Rhodanin-3-essigsäure, 1-Methyl-2-chinaldinium-iodid, 1-Ethyl-2-chinaldinium-iodid, 1,4-Dimethylchinolinium-iodid, Barbitursäure, Thiobarbitursäure, 1,3-Dimethylthiobarbitursäure, 1,3-Diethylthiobarbitursäure, Diethylthiobarbitursäure, Oxindol, 3-Indoxylacetat, Cumaranon, 1-Methyl-3-phenyl-2-pyrazolinon, Indan-1,3-dion, Indan-1-on und 1-Dicyanmethylenindan.

Die Verbindungen der Komponente B werden besonders bevorzugt ausgewählt aus der Gruppe bestehend aus N-(2-Hydroxyethyl)-N-ethyl-, 2-Chlor-p-phenylendiamin, N,N-Bis-(2-hydroxyethyl)-p-phenylendiamin, 4-Aminophenol, p-Phenylendiamin, 2-(2,5-Diaminophenyl)-ethanol, 2,5-Diaminotoluol, 3,4-Methylendioxyanilin, 2-Amino-4-(2-hydroxyethylamino)-anisol, 2-(2,4-Diaminophenoxy)-ethanol, 3-Amino-2,4-dichlor-, 2-Methyl-5-amino-, 3-Methyl-4-amino-, 2-Methyl-5-(2-hydroxyethylamino)-, 2-Methyl-5-amino-4-chlor-, 6-Methyl-3-amino-2-chlor-, 2-Aminomethyl-4-aminophenol, 2-Diethylaminomethyl-4-aminophenol, 2-Dimethylaminomethyl-4-aminophenol, 2,6-Dichlor-4-aminophenol, 2-Hydroxymethyl-4-aminophenol, 3,4-Methylendioxyphenol, 3,4-Diaminobenzoesäure, 2,5-Diamino-, 2-Dimethylamino-5-amino-, 3-Amino-2-methylamino-6-methoxy-, 2,3-Diamino-6-methoxy-, 3,5-Diamino-2,6-dimethoxy-, 2,6-Dihydroxy-3,4-dimethylpyridin, 2-Hydroxy-4,5,6-triami-no-, 4-Hydroxy-2,5,6-triamino-, 2,4,5,6-Tetraamino-, 2-Methylamino-4,5,6-triaminopyrimidin, 3,5-Diaminopyrazol, 3-Amino-5-hydroxypyrazol, 4,5-Diamino-1-(2-hydroxyethyl)-pyrazol, 5,6-Dihydroxyindol und 5,6-Dihydroxyindolin, β-Alanin, L-Prolin, L-Lysin, DL-Tyrosin sowie deren mit vorzugsweise anorganischen Säuren gebildeten physiologisch verträglichen Salze.

Die voranstehend genannten Verbindungen der Komponente B können in einer Menge von jeweils 0,03 bis 65, insbesondere 1 bis 40 mmol, jeweils bezogen auf 100 g des gesamten Färbemittels, eingesetzt werden. Weiterhin kann es erfindungsgemäß besonders bevorzugt sein, die Verbindung der Formel la oder Ib oder Ic und die Verbindung der Komponente B im molaren Mengenverhältnis von 2: 1 bis 1 : 2, insbesondere etwa äquimolar, einzusetzen. Beim Einsatz von Verbindungen der Komponente B, die eine oder mehrere Aminogruppen enthalten, werden die Verbindungen der Formel Ia oder Ib oder Ic einerseits und die Verbindungen der Komponente B andererseits bevorzugt in solchen Mengenverhältnissen eingesetzt, dass die Summe der Zahl der Carbonylgruppen der Verbindungen der Formel la oder Ib oder Ic und die Summe der Zahl der Aminogruppen der Verbindungen der Komponente B im Verhältnis 2 : 1 bis 1 : 2 stehen.

Zur Erlangung weiterer und intensiverer Ausfärbungen können die erfindungsgemäßen Mittel zusätzlich Farbverstärker enthalten. Die Farbverstärker sind vorzugsweise ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazidin, deren Derivate sowie deren physiologisch verträglichen Salzen.

Die voranstehend genannten Farbverstärker können in einer Menge von jeweils 0,03 bis 65, insbesondere 1 bis 40 mmol, jeweils bezogen auf 100 g des gesamten Färbemittels, eingesetzt werden.

Auf die Anwesenheit von Oxidationsmitteln, z. B. H₂O₂, kann dabei verzichtet werden. Es kann jedoch u. U. wünschenswert sein, den erfindungsgemäßen Mitteln zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungsmischung eingesetzt. Ein für menschliches Haar bevorzugtes Oxidationsmittel ist H₂O₂.

In einer bevorzugten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe, z. B. aus der Gruppe der Nitrophenylendiamine, Nitroaminophenole, Anthrachinone oder Indophenole, wie z. B. die unter den internationalen Bezeichnungen bzw. Handelsnamen HC Yellow 2, HC Yellow 4, HC Yellow 6, Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16 und Basic Brown 17 bekannten Verbindungen sowie Pikraminsäure 2-Amino-6-chloro-4-nitrophenol, 4-Amino-2-nitrodiphenylamin-2'-carbonsäure, 6-Nitro-1,2,3,4-tetrahydrochinoxalin, 4-N-Ethyl-1,4-bis-(2'-hydroxyethylamino)-2-nitrobenzol-hydrochlorid und 1-Methyl-3-nitro-4-(2'-hydroxyethyl)-aminobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die fakultativ enthaltenen direktziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z. B. Cremes, Emulsionen, Gele oder auch tensidhaltige schäumende Lösungen wie z. B. Shampoos oder andere Zubereitungen, die für die Anwendung auf den keratinhaltigen Fasern geeignet sind. Falls erforderlich ist es auch möglich, die Färbemittel in wasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobemsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex^{®} vertriebenen Methylhydroxyalkyldialkoyloxyalkylammoniummethosulfate.

Ein Beispiel für ein als kationisches Tensid einsetzbares quaternäres Zuckerderivat stellt das Handelsprodukt Glucquat^{®}100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und VinylpyrrolidonNinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quatemierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, VinylpyrrolidonNinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammonium- oder Metallsalze zuzugeben. Geeignete Metallsalze sind z. B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65, insbesondere von 1 bis 40, mmol bezogen auf 100 g des gesamten Färbemittels, enthalten.

Der pH-Wert der gebrauchsfertigen Färbezubereitungen liegt üblicherweise zwischen 2 und 11, vorzugsweise zwischen 5 und 10.

Ein weiterer Gegenstand der vorliegenden Erfindung betrifft die Verwendung von Chinoxalinderivaten mit der Formel la oder Ib oder Ic, 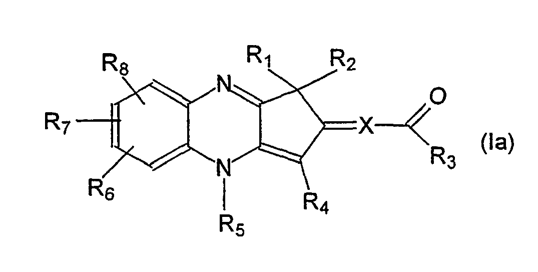 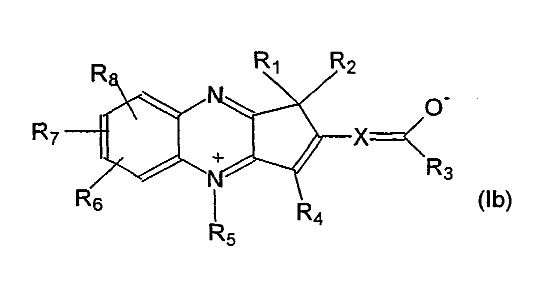 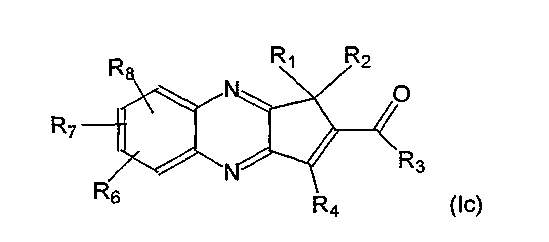 worin R¹, R², R³, R⁴, R⁵, R⁶, R⁷, R⁸ und X wie oben definiert sind,
als eine färbende Komponente in Oxidationshaarfärbemitteln.

Noch ein weiterer Gegenstand der vorliegenden Erfindung betrifft ein Verfahren zum Färben von keratinhaltigen Fasern, insbesondere menschlichen Haaren, worin ein Färbemittel, enthaltend
A) mindestens ein Chinoxalinderivat mit der Formel Ia oder Ib oder Ic, 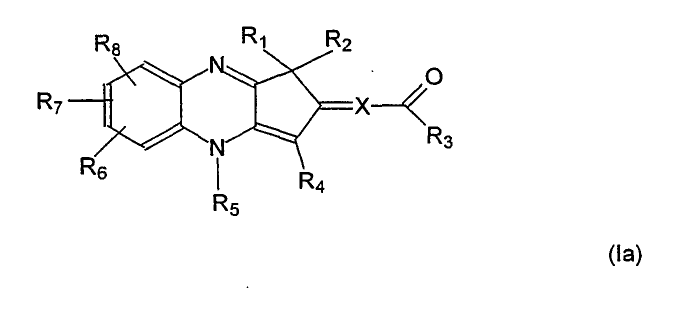 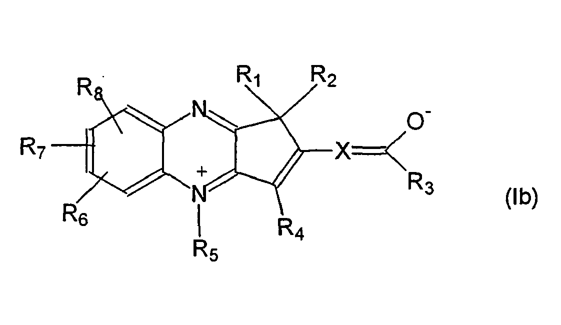 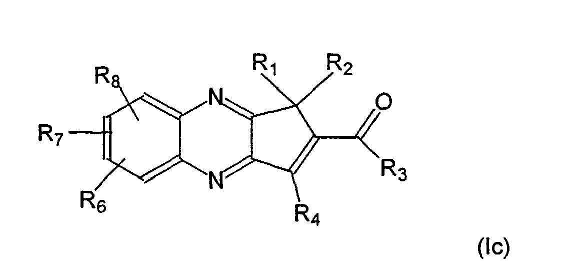 worin R¹, R², R³, R⁴, R⁵, R⁶, R⁷, R⁸ und X wie oben definiert sind,
   und
B) mindestens eine Verbindung mit primärer oder sekundärer Aminogruppe oder Hydroxygruppe, ausgewählt aus primären oder sekundären aliphatischen oder aromatischen Aminen, stickstoffhaltigen heterocyclischen Verbindungen, aromatischen Hydroxyverbindungen, Aminosäuren, aus 2 bis 9 Aminosäuren aufgebauten Oligopeptiden und/oder mindestens eine CH-aktive Verbindung,
sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

Die Chinoxalinderivate der Formel la oder Ib oder Ic und die Verbindungen der Komponente B können entweder gleichzeitig auf das Haar aufgebracht werden oder aber auch nacheinander, wobei es unerheblich ist, welche der beiden Komponenten zuerst aufgetragen wird. Die fakultativ enthaltenen Ammonium- oder Metallsalze können dabei der ersten oder der zweiten Komponente zugesetzt werden. Zwischen dem Auftragen der ersten und der zweiten Komponente können bis zu 30 Minuten Zeitabstand liegen. Auch eine Vorbehandlung der Fasern mit der Salzlösung ist möglich.

Die Chinoxalinderivate der Formel la oder Ib oder Ic und die Verbindungen der Komponente B können entweder getrennt oder zusammen gelagert werden, entweder in einer flüssigen bis pastösen Zubereitung (wässrig oder wasserfrei) oder als trockenes Pulver. Werden die Komponenten in einer flüssigen Zubereitung zusammen gelagert, so sollte diese zur Verminderung einer Reaktion der Komponenten weitgehend wasserfrei sein. Bei der getrennten Lagerung werden die reaktiven Komponenten erst unmittelbar vor der Anwendung miteinander innig vermischt. Bei der trockenen Lagerung wird vor der Anwendung üblicherweise eine definierte Menge warmen (30 bis 80°C) Wassers hinzugefügt und eine homogene Mischung hergestellt.

### Beispiele

### Herstellung der eingesetzten Färbekomponenten

### Beispiel 1: Darstellung von 2-Formyl-1,1,3-trimethyl-1H-cyclopenta(b)chinoxalin (1,1,3-Trimethylcyclo-2-penten-(1,2-b)chinoxalin-2-carbaldehyd)

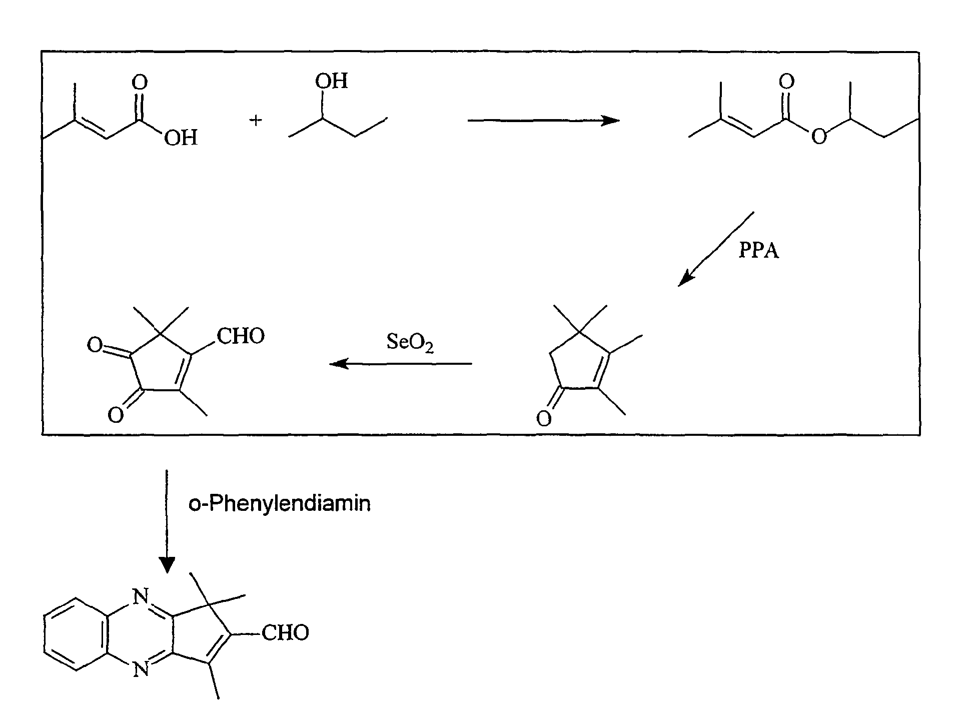

### 1. Stufe: Darstellung von 3-Methyl-2-butensäure-(2'-butylester)

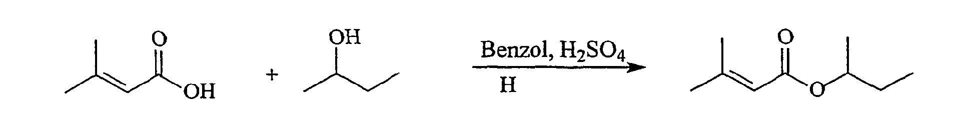

In einem mit Wasserabscheider versehenen 2 I Einhalskolben wurden 200 g (2,0 mol) käufliche 3,3-Dimethylacrylsäure und 148 g (2,0 mol) Isobutanol in 540 ml Benzol gelöst, 7 ml konzentrierte Schwefelsäure zugegeben und solange unter Rückfluß erhitzt, bis sich kein Reaktionswasser mehr abschied (ca. 72 Stunden). Das erkaltete Reaktionsgemisch wurde zweimal mit 100 ml gesättigter Natriumchloridlösung und einmal mit 100 ml gesättigter Natriumhydrogencarbonatlösung gewaschen. Nach dem Trocknen über Magnesiumsulfat wurde das Lösungsmittel am Rotationsverdampfer entfernt und der hellgelbe Rückstand im Wasserstrahlvakuum destilliert. Man erhielt das Produkt bei einer Übergangstemperatur von 68°C (13 Torr) als farblose Flüssigkeit.

| | |
|---|---|
| Ausbeute: | 235,3 g (1,5 mol) 77% der Theorie |
| Sdp. | 68°C (13 Torr) |
| n_{D}²⁰: | 1,4383 |

### 2. Stufe: Darstellung von 2,3,4,4-Tetramethyl-cyclopent-2-en-1-on

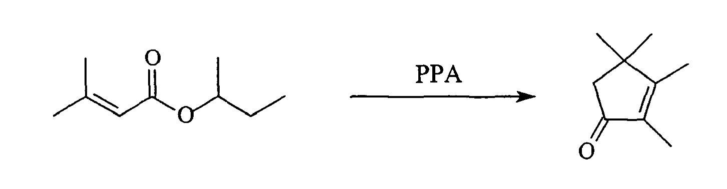

Unter kräftigem Rühren wurden 1 kg Polyphosphorsäure (PPA) in einem mit Rückflußkühler, KPG-Rührer mit Stahlrührwelle und Tropftrichter bestückten 2 I Dreihalskolben auf 120°C Ölbadtemperatur erwärmt. Innerhalb von 30 Minuten wurden 200,0 g (1,28 mol) 3-Methyl-2-butensäure-(2'-butylester) hinzugetropft und anschließend noch eine Stunde nachgerührt. Das Heizbad wurde entfernt und der noch heiße Kolben bei abgestelltem Rührer mit gestoßenem Eis auf 2/3 seines Volumens aufgefüllt. Es wurde wieder angerührt und mit Eiswasser auf ca. 1,7 I aufgefüllt. Die Lösung wurde mit festem Ammoniumchlorid gesättigt und anschließend fünfmal mit je 200 ml Diethylether extrahiert. Die vereinigten Etherphasen wurden mit je 200 ml zehnprozentiger Natriumhydrogencarbonatlösung und gesättigter Natriumchloridlösung gewaschen. Nach Trocknen über Magnesiumsulfat und Entfernen des Lösungsmittels am Rotationsverdamfer wurde der dunkelbraune Rückstand fraktioniert destilliert und das Produkt als hellgelbe Flüssigkeit bei einer Übergangstemperatur von 70 °C (5 Torr) erhalten.

| | |
|---|---|
| Ausbeute: | 87,4 g (0,63 mol), 49% der Theorie |
| Sdp.: | 70°C (5 Torr) |
| n_{D}²⁰: | 1,4727 |

### 3. Stufe: Darstellung von 4-Formyl-3,5,5-trimethylcyclopent-3-en-1,2-dion (2,5,5-Trimethyl-3,4-dioxocyclopent-1-en-1-carbaldehyd)

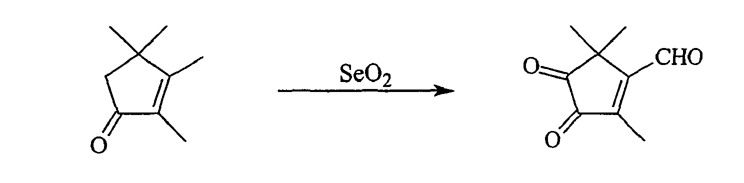

In einem 500 ml Einhalskolben wurde 101,7 g (0,92 mol) Selendioxid in 230 ml Eisessig suspendiert und auf ca. 60°C erwärmt. Unter Rühren wurden 45,2 g (0,33 mol) 2,3,4,4-Tetramethyl-cyclopent-2-en-1-on hinzugegeben, und es wurde vier Stunden unter Rückfluß gekocht. Man konnte hierbei eine rasche Farbvertiefung von gelb über rot nach dunkelbraun beobachten. Danach wurde die Mischung auf 0°C gekühlt und über einen Büchnertrichter vom ausgefallenen Selen bzw. vom nicht umgesetzten Selendioxid abfiltriert. Anschließend wurde das Lösungsmittel am Rotationsverdampfer entfernt und durch ca. 300 ml Diethylether ersetzt. Es wurde 30 Minuten unter heftigem Rühren refluxiert, nach dem Erkalten erneut filtriert und der Diethylether am Rotationsverdampfer abdestilliert. Der Rückstand wurde über eine 10 cm Vigreuxkolonne im Ölpumpenvakuum fraktioniert destilliert. Das bei Übergangstemperaturen von 87 - 92 °C (0,05 mbar) erhaltene dunkelrote, hochviskose Produkt kristallisierte oftmals bereits am Kühlfinger in Form dunkelroter Kristalle aus. Anschließend wurde in wenig Diethylether umkristallisiert.

| | |
|---|---|
| Ausbeute: | 24,4 g (0,177 mol), 54% der Theorie |
| Sdp.: | 87 - 92°C (0,05 mbar) |

### 4. Stufe: Darstellung von 2-Formyl-1,1,3-trimethyl-1H-cyclopenta(b)chinoxalin (1,1,3-Trimethylcyclo-2-penten-(1,2-b)chinoxalin-2-carbaldehyd)

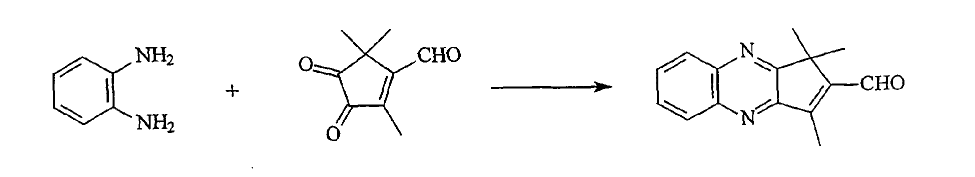

In einem 250 ml Kolben wurden 3,0 g (18 mmol) 4-Formyl-3,5,5-trimethylcyclopent-3-en-1,2-dion und 1,9 g (18 mmol) o-Phenylendiamin in 150 ml Chloroform gelöst und zwei Stunden unter Rückfluß gekocht. Nach dem Trocknen über Magnesiumsulfat wurde das Lösungsmittel am Rotationsverdampfer abgezogen. Es wurde eine Säulenchromatographie an Kieselgel mit Dichlormethan als Laufmittel durchgeführt; man erhielt das Produkt als zweite, gelbe Fraktion. Nicht umgesetzter Diketoaldehyd wurde vorher als erste Fraktion eluiert. Eine Umkristallisation in Tetrahydrofuran lieferte das Produkt in Form von gelben Nadeln.

| | |
|---|---|
| Ausbeute: | 1,1 g (4,6 mmol), 26% der Theorie |

### Beispiel 2: Darstellung von (1,1,3-Trimethyl-4-methyl-1H-cyclopenta(b)-chinoxalin-2-yliden)-acetaldehyd

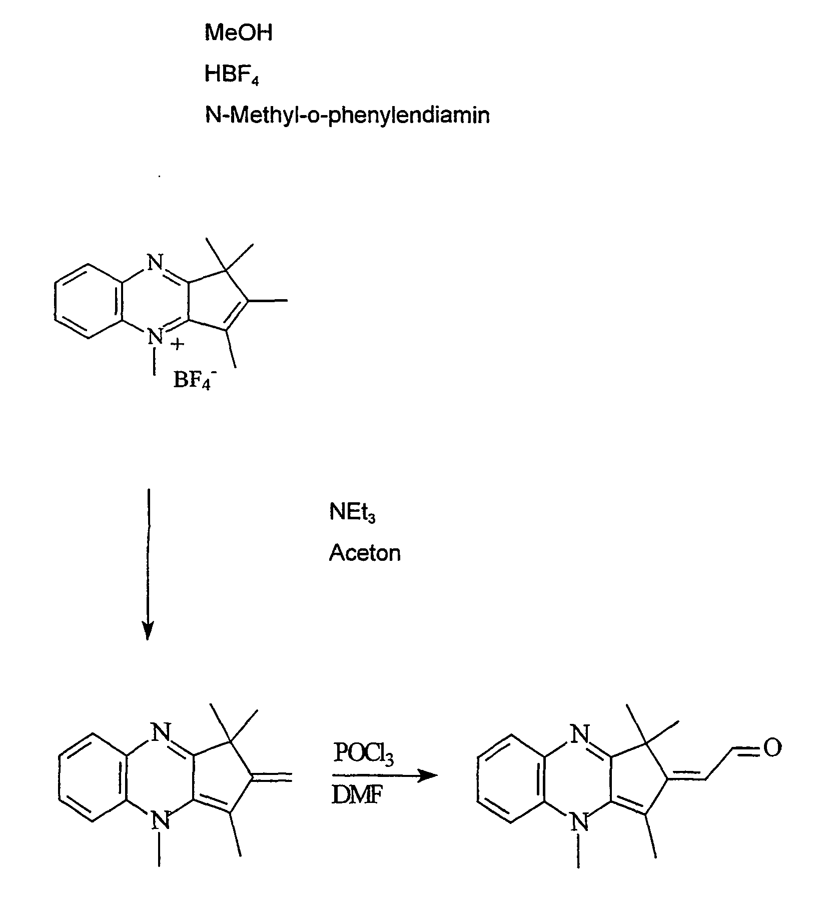

Die beiden ersten Stufen wurden analog Beispiel 1 durchgeführt.

### Stufe 3: Darstellung von 5-Acetoxy-2,3,4,4-tetramethyl-2-cyclopenten-1-on

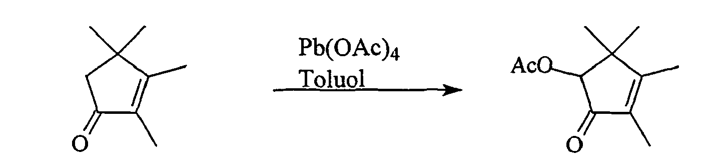

Unter Stickstoffatmosphäre wurden in einem 2 I Dreihalskolben 385 g (0,87 mol) Blei(IV)-acetat in 1,3 I absolutem Toluol vorgelegt und unter Rühren mit einem KPG-Rührer erwärmt. Bei einer Temperatur von 80°C tropfte man nun rasch 100 g (0,73mol) 2,3,4,4-Tetramethyl-cyclopent-2-en-1-on zum Oxidationsmittel und erhitzte anschließend sechs Stunden unter Rückfluß. Vom voluminös ausfallenden Blei(II)acetat filtrierte man noch heiß über einen Büchnertrichter ab, und der Filterrückstand wurde bis zur Farblosigkeit mehrfach mit Diethylether ausgewaschen. Die vereinigten organischen Filtrate wurden zweimal mit je 200 ml gesättigter Natriumchloridlösung gewaschen, die wässrige Phase wurde zweimal mit je 200 ml Diethylether extrahiert. Nach Trocknen über Magnesiumsulfat wurde das Lösungsmittel am Rotationsverdampfer abgezogen und der ölige Rückstand im Ölpumpenvakuum über eine 30 cm Vigreuxkolonne destilliert. Bei Übergangstemperaturen bis zu 60°C (0,8 Torr) wurde nicht umgesetztes Cyclopentenon zurückgewonnen. Man erhielt das Acyloinacetat bei einer Übergangstemperatur von 93 - 96°C (1 Torr).

| | |
|---|---|
| Ausbeute: | 72 g , 49% der Theorie |

### 4. Stufe: Darstellung von 5-Hydroxy-2,3,4,4-tetramethyl-2-cyclopenten-1-on

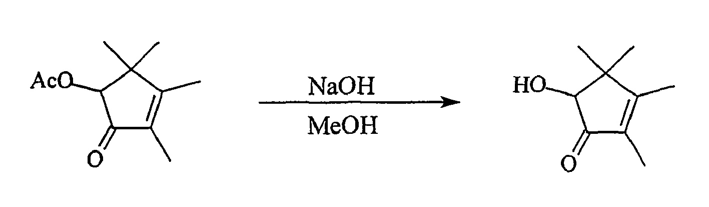

Eine Lösung von 60,0 g (0,30 mol) 5-Acetoxy-2,3,4,4-tetramethyl-2-cyclopenten-1-on in 400 ml Methanol wurde unter Stickstoffatmosphäre in einem 2 I Dreihalskolben vorgelegt und mit Eis gekühlt. In der Kälte tropfte man innerhalb von 20 Minuten 300 ml 1 N Natriumhydroxidlösung zu und rührte das Reaktionsgemisch weitere zwei Stunden bei Raumtemperatur. Durch Zugabe von 70 ml 0,5 N Schwefelsäurelösung wurde das Reaktionsgemisch auf einen pH-Wert von ca. 6 gebracht. Es wurde mit 500 ml gesättigter Natriumchloridlösung versetzt und schließlich viermal mit je 300 ml Diethylether extrahiert. Die vereinigten organischen Phasen wurden mit 100 ml gesättigter Natriumchloridlösung gewaschen und über Magnesiumsulfat getrocknet. Nach Abziehen des Lösungsmittels erhielt man ein braungelbes Öl, welches im Ölpumpenvakuum über eine 30 cm Vigreuxkolonne destilliert wurde. Bei einer Übergangstemperatur von 62 - 64°C (0,4 Torr) erhielt man das Produkt als gelbes Öl.

| | |
|---|---|
| Ausbeute: | 32 g (207 mmol), 68% der Theorie |
| Sdp.: | 62 - 64°C (0,4 Torr) |

### 5. Stufe: Darstellung von 3,4,5,5-Tetramethylcyclopent-3-en-1,2-dion

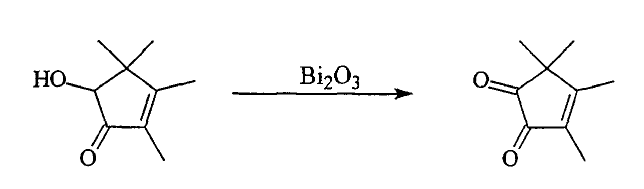

Eine Lösung von 30 g (0,20 mol) 5-Hydroxy-2,3,4,4-tetramethyl-2-cyclopenten-1-on in 200 ml Eisessig wurde in einem 500 ml Rundkolben auf 80°C erwärmt, bevor man sie unter Rühren mit 65 g (170 mmol) Wismut(III)oxid versetzte. Unter Stickstoffatmosphäre wurde das Reaktionsgemisch nun weitere acht Stunden unter Rückfluß erhitzt. Die mit der Abscheidung elementaren Wismuts einhergehende Trübung des Ansatzes zeigte den Umsatz des Oxidans an. Nach Beendigung der Reaktion wurde noch heiß vom ausgefallenen grauen Niederschlag abgesaugt und der Filterrückstand mit Eisessig nachgewaschen. Die Filtrate wurden am Rotationsverdampfer scharf eingeengt; der anfallende ölige Rückstand wurde mit 700 ml Diethylether aufgekocht. Nach Abnutschen vom unlöslichen Wismut(III)acetat wurde das Filtrat erneut vom Lösungsmittel befreit und im Ölpumpenvakuum fraktioniert destilliert. Bei einer Übergangstemperatur von 73°C (0,6 Torr) erhielt man das Diketon als intensiv oranges Öl, welches teilweise schon am Kühlfinger in seiner farblosen Enolform auskristallisierte.

| | |
|---|---|
| Ausbeute: | 25 g (164 mmol), 84% der Theorie |
| Sdp.: | 73°C (0,6 Torr) |

### 6. Stufe: Darstellung von 1,1,2,3,4-Pentamethyl-1H-cyclopenta(b)chinoxalinium-tetrafluoroborat

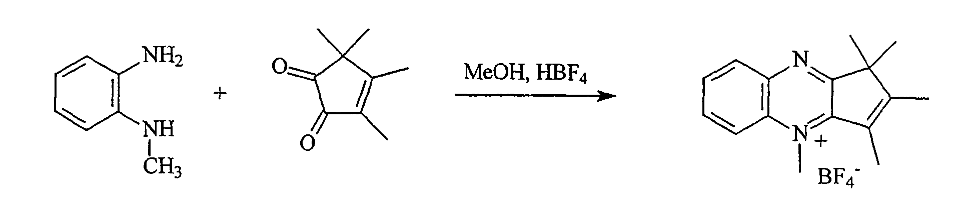

In einem 250 ml Kolben wurden 4,00 g (32,7mmol) N-Methyl-o-phenylendiamin und 4,98 g (32,7 mmol) 3,4,5,5-Tetramethyl-cyclopent-2-en-1,2-dion in 100 ml Methanol gelöst und mit 25 ml 32%iger Tetrafluorborsäure versetzt. Das Reaktionsgemisch wurde 6 Stunden bei Raumtemperatur gerührt. Dann wurde die Reaktionslösung in eiskalten Diethylether eingetropft, wobei ein beiger Feststoff ausfiel. Der Feststoff wurde nochmals in Methanol aufgenommen und durch Eintropfen in kalten Diethylether ausgefällt.
Ausbeute: 4,20 g (12,9 mmol), 39% der Theorie

### 7. Stufe: Darstellung von 1,1,3-Trimethyl-2-methylen-4-methyl-1H-2,4-dihydro-cyclopenta(b)-chinoxalin

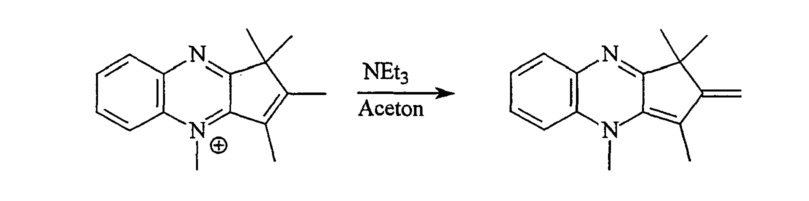

In einem 250 ml Kolben wurden 6,21 g (19 mmol) 1,1,2,3,4-Pentamethyl-1H-cyclopenta(b)chinoxaliniumtetrafluoroborat in 80 ml Aceton unter Schutzgasatmosphäre suspendiert. Das Edukt löste sich mit grünlich-blauer Farbe, welche nach Zugabe von 5 ml Triethylamin in einen gelblichen Farbton umschlug. Man rührte noch eine Stunde bei Raumtemperatur und gab dann 250 ml destilliertes Wasser zu der Reaktionslösung. Nach dreimaliger Extraktion mit je 100 ml Diethylether wurde kurz über Magnesiumsulfat getrocknet, das Lösungsmittel am Rotationsverdampfer entfernt und das Rohprodukt infolge der Luft- und Lichtinstabilität des Enamins sofort weiter umgesetzt.

| | |
|---|---|
| Ausbeute: | 3,0 g (10 mmol) 55% der Theorie; |

### Stufe 8: Darstellung von (1,1,3-Trimethyl-4-methyl-1H-cyclopenta(b)-chinoxalin-2-yliden)-acetaldehyd

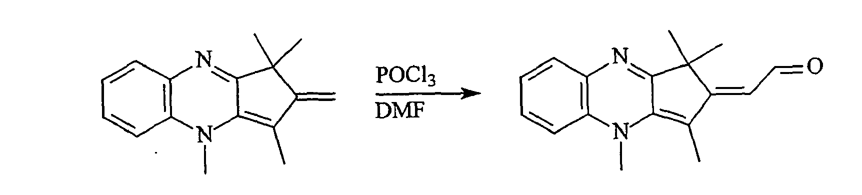

Es wurden 3,00 g (10 mmol) 1,1,3-Trimethyl-2-methylen-4-methyl-1H-2,4-dihydro-cyclopenta-chinoxalin in 85 ml absolutem N,N-Dimethylformamid gelöst. Unter Rühren tropfte man zu dieser Lösung eine unter Eiskühlung bereitete Lösung aus 6,15g (43 mmol) Phosphoroxichlorid und 11,25 g (155 mmol) N,N-Dimethylformamid hinzu. Hierbei färbte sich der Ansatz intensiv rot. Das Eisbad wurde entfernt und der Ansatz noch zwei bis drei Stunden bei Raumtemperatur gerührt. Anschließend wurde das Reaktionsgemisch auf Eis gegossen und mit 10%iger Natriumhydroxidlösung alkalisch gestellt; hierbei fiel ein orange-brauner Niederschlag aus. Eine säulenchromatographische Aufreinigung an Kieselgel mit dem Laufmittelgemisch Essigsäureethylester/Hexan 2:1 ergab einen orangen, metallisch glänzenden Feststoff, der als intensiv orange Fraktion von der Säule eluiert wurde.

| | |
|---|---|
| Ausbeute: | 1,31 g (4 mmol), 40 % der Theorie |

### Herstellung der Färbegele

Die angegebene Menge Chinoxalinderivat mit der Formel la oder Ib oder Ic wurde gegebenenfalls unter Zusatz von Ammoniak in Wasser gelöst und die Lösung wurde mit 2 % Natrosol^{®} 250 HR verdickt. Die Zusammensetzungen der Färbegele sind in der folgenden Tabelle 1 wiedergegeben.

### Zusammensetzung der Färbegele (Tab. 1)

| Komponenten | Färbegel 1 Menge (g) | Färbegel 2 Menge (g) |
|---|---|---|
| 1,1,3-Trimethylcyclo-2-penten(1,2-b)chinoxalin-2-carbaldehyd | 4,4 | - |
| (1,1,3-Trimethyl-4-methyl-1 H-cyclopenta(b)-chinoxalin-2-yliden)-acetaldehyd | - | 4,8 |
| Natrosol^{®} 250 HR | 2,0 | 2,0 |
| Wasser, dest. | ad 100 | ad 100 |

### Herstellung der Farbcremes

Texapon^{®}NSO, Dehyton^{®}K, Hydrenol^{®}D, Lorol^{®} techn. und Eumulgin^{®}B2 wurden unter Zusatz von ca. 10 g Wasser bei 80°C emulgiert. Separat wurden die Farbstoffe in Wasser gelöst, mit Ammoniak oder NaOH sowie Ascorbinsäure und Natriumsulfit versetzt und die Lösung mit der Emulsion vermischt. Die einzelnen Zusammensetzungen sind in Tabelle 2 wiedergegeben.

### Legende zu den Tabellen 1 und 2

| | |
|---|---|
| Natrosol^{®}250 HR = | Hydroxyethylcellulose |
| Texapon^{®}NSO = | Na-Laurylethersulfat (26,5-27%-ig) |
| Dehyton^{®}K = | Cocoamidopropylbetain (29-32%-ig) |
| Hydrenol^{®}D = | Cetearylalkohol |
| Lorol^{®}techn. = | Kokosfettalkohol |
| Eumulgin^{®}B2 = | Ceteareth-20 |

Das Färbegel 1 oder 2 wurde mit einer der Farbcremes aus Tabelle 2 im Gewichtsverhältnis 1 : 1 vermischt, mit Ammoniak oder Weinsäure auf den in Tabelle 3 angegebenen pH-Wert eingestellt und auf Haarsträhnen (Kerling, naturweiß) appliziert. die Einwirkzeit betrug 30 Minuten bei 32°C.

Es wurden die in Tabelle 3 genannte Farbergebnisse gemäß Deutschem Farbatlas erhalten.

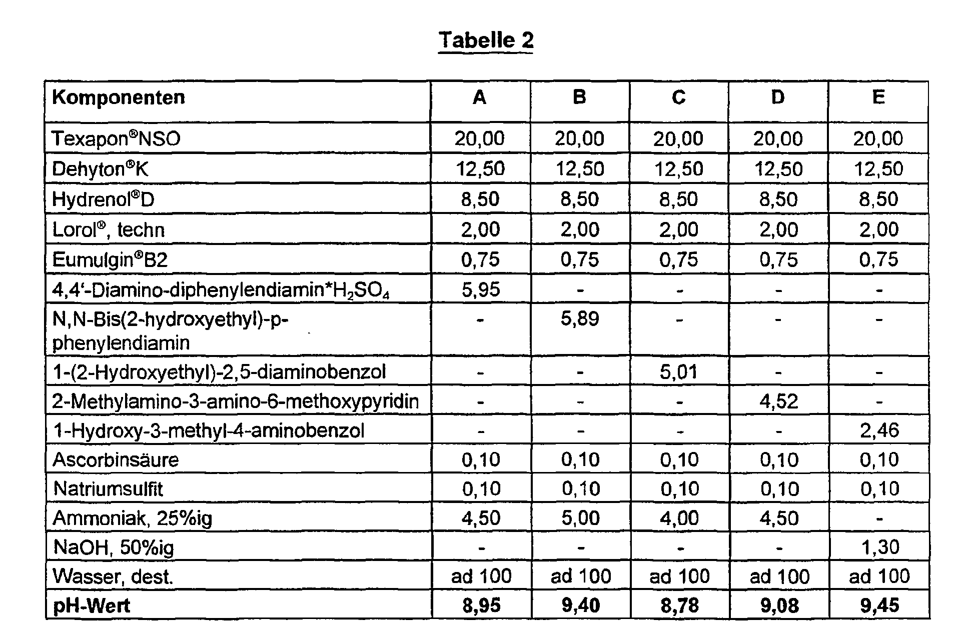

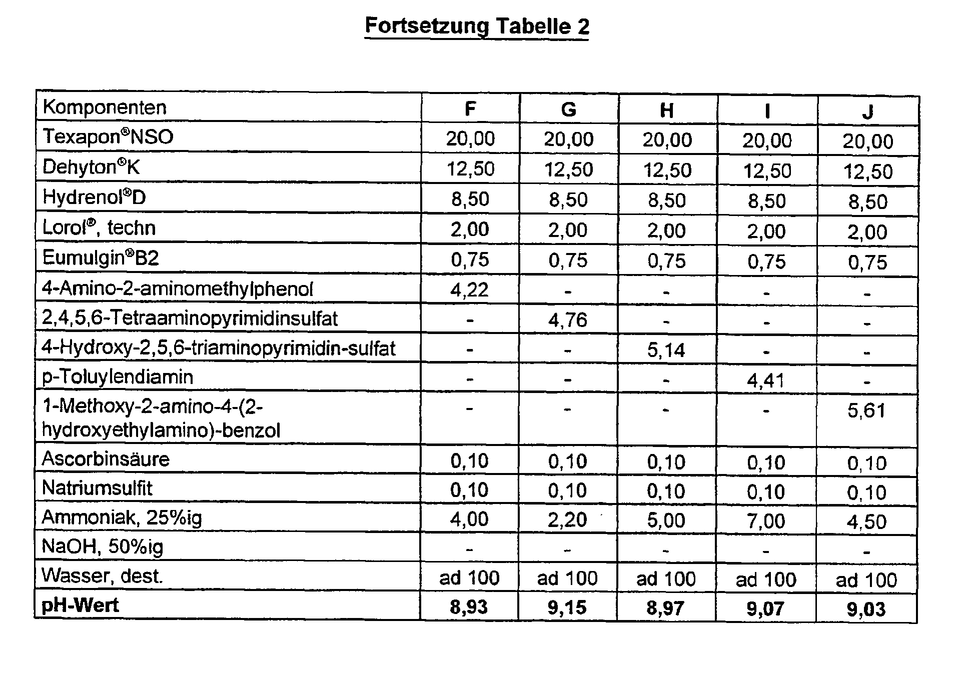

**Tabelle 3**

| **Färbegel** | **Farbcreme** | **Misch-pH** | **Farbergebnis** |
|---|---|---|---|
| 1 | A | 8,5 | graubraun |
| 1 | B | 8,9 | hellbraun |
| 1 | C | 9,1 | honiggelb |
| 1 | D | 8,8 | goldbraun |
| 1 | J | 8,8 | goldbraun |
| 2 | A | 6,5 | currygelb |
| 2 | A | 9,4 | mattgrün |
| 2 | B | 5,9 | tiefblau |
| 2 | B | 8,6 | blaßtürkis |
| 2 | C | 5,3 | dunkelblau |
| 2 | C | 8,9 | blau |
| 2 | E | 9,3 | graurot |
| 2 | E | 6,6 | purpur |
| 2 | F | 9,7 | tiefviolett |
| 2 | F | 6,2 | rotviolett |
| 2 | G | 10,3 | hellrot |
| 2 | H | 9,2 | graumagenta |
| 2 | H | 5,3 | graumagenta |
| 2 | I | 9,6 | grauviolett |
| 2 | I | 6,4 | grauviolett |

## Patentansprüche

1. Mittel zum Färben von keratinhaltigen Fasern, insbesondere menschlichen Haaren, enthaltend als färbende Komponente mindestens ein Chinoxalinderivat mit der Formel Ia oder Ib oder Ic, 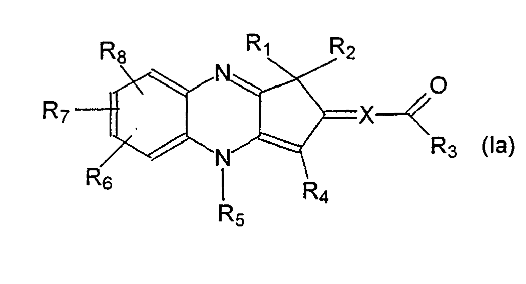 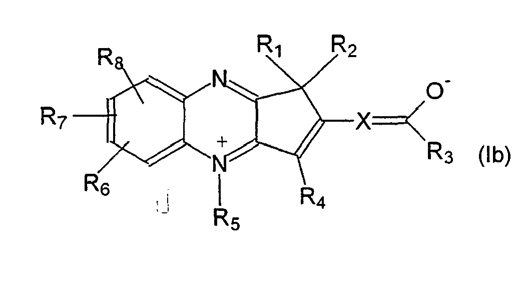 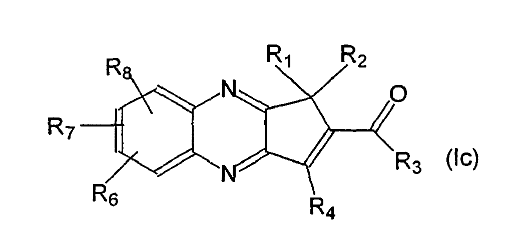 in der bedeuten
R¹, R², R³, R⁴ unabhängig voneinander ein Wasserstoffatom oder eine C₁-C₄₋Alkylgruppe,
R⁵ ein Wasserstoffatom, eine C₁-C₆-Alkyl- oder Aralkyl-, Aryl-, C₁-C₄-Alkenyl-, Hydroxyalkyl-, Carboxyalkylgruppe,
R⁶, R⁷, R⁸ unabhängig voneinander ein Wasserstoff-, Halogenatom, eine C₁-C₄-Alkyl-, C₁-C₄-Alkoxy-, Nitro-, Amino-, Alkylamino-, Hydroxy-, Carboxy-, Sulfo-, Thiol-, oder C₁-C₄-Alkylthiogruppe, wobei je zwei Reste auch gemeinsam einen Ring bilden können,
wobei -X= bzw. =X- für -CH=, =CH- steht.

2. Mittel nach Anspruch 1, **dadurch gekennzeichnet, dass** als Verbindungen mit der Formel Ia oder Ib oder Ic 1,1,3-Trimethyl-1H-cyclopenta[b]chinoxalin-2-carbaldehyd, 6(7)-Chlor-, 6(7)-Fluor-, 6(7)-Brom-, 6(7)-Methyl-, 6(7)-Nitro-, 6,7-Dichlor-, 6,7-Methylendioxy-1,1,3-trimethyl-1H-cyclopenta[b]chinoxalin-2-carbaldehyd, 1,1,3,6(7)-Tetramethyl-, 1,1,3,6,7-Pentamethyl-1H-cyclopenta[b]chinoxalin-2-carbaldehyd, 1,1,3-Trimethyl-1H-benzo[g]cyclopenta[b]chinoxalin-2-carbaldehyd, 2,4-Dihydro-1,1,3,4-tetramethyl-1H-cyclopenta[b]chinoxalin-2-yliden-acetaldehyd, 2,4-Dihydro-4-phenyl-1,1,3-trimethyl-1H-cyclopenta[b]chinoxalin-2-yliden-acetaldehyd, 6,7-Dichlor-2,4-dihydro-1,1,3,4-tetramethyl-1H-cyclopenta[b]chinoxalin-2-yliden-acetaldehyd, 7-Chlor-2,4-dihydro-4-(2-propyl)-1,1,3-trimethyl-1H-cyclopenta[b]chinoxalin-2-yliden-acetaldehyd, 2,4-Dihydro-6,7-methylendioxy-1,1,3,4-tetramethyl-1H-cyclopenta[b]chinoxalin-2-yliden-acetaldehyd sowie deren beliebigen Gemische eingesetzt werden.

3. Mittel nach einem der Ansprüche 1 bis 2, **dadurch gekennzeichnet, dass** die Chinoxalinderivate der Formel la oder Ib oder Ic in einer Menge von 0,03 bis 65 mmol, insbesondere von 1 bis 40 mmol, bezogen auf 100 g des gesamten Färbemittels, enthalten sind.

4. Mittel nach einem der Ansprüche 1 bis 3, **dadurch gekennzeichnet, dass** es zusätzlich mindestens eine Verbindung mit primärer oder sekundärer Aminogruppe oder Hydroxygruppe, ausgewählt aus primären oder sekundären aromatischen Aminen, stickstoffhaltigen heterocyclischen Verbindungen und aromatischen Hydroxyverbindungen, Aminosäuren, aus 2 bis 9 Aminosäuren aufgebauten Oligopeptiden und/oder mindestens eine CH-aktive Verbindung, enthält.

5. Mittel nach Anspruch 4, **dadurch gekennzeichnet, dass** die weitere Verbindung ausgewählt ist aus
primären oder sekundären Aminen aus der Gruppe, bestehend aus N-(2-Hydroxyethyl)-N-ethyl-, N-(2-Methoxyethyl-), 2,3-, 2,4-, 2,5-Dichlor-p-phenylendiamin, 2-Chlor-p-phenylendiamin, N,N-Bis-(2-hydroxyethyl)-p-phenylendiamin, 2,5-Dihydroxy-4-morpholinoanilin-dihydrobromid, 2-, 3-, 4-Aminophenol, o-, m-, p-Phenylendiamin, 2,4-Diaminophenoxyethanol, 2-(2,5-Diaminophenyl)-ethanol, 2,5-Diaminotoluol, - phenol, -phenethol, 4-Methylamino-, 3-Amino-4-(2'-hydroxyethyloxy)-, 3,4-Methylendiamino-, 3,4-Methylendioxyanilin, 3-Amino-2,4-dichlor-, 4-Methylamino-, 2-Methyl-5-amino-, 3-Methyl-4-amino-, 2-Methyl-5-(2-hydroxyethylamino)-, 2-Methyl-5-amino-4-chlor-, 6-Methyl-3-amino-2-chlor-, 5-(2-Hydroxyethylamino)-4-methoxy-2-methyl-, 4-Amino-2-aminomethyl-phenol, 2-Hydroxymethyl-4-aminophenol, 2-Diethylaminomethyl-4-aminophenol, 2-Dimethylaminomethyl-4-aminophenol, 2,6-Dichlor-4-aminophenol, 1,3-Diamino-2,4-dimethoxybenzol, 2-, 3-, 4-Aminobenzoesäure, -phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxybenzoesäure, 2-, 3-, 4-Aminobenzolsulfonsäure, 3-Amino-4-hydroxybenzolsulfonsäure, 4-Amino-3-hydroxynaphthalin-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol-tetrahydrochlorid, 2,4,5-Triaminophenol-trihydrochlorid, Pentaaminobenzolpentahydrochlorid, Hexaaminobenzol-hexahydrochlorid, 2,4,6-Triaminoresorcin-trihydrochlorid, 4,5-Diaminobrenzcatechinsulfat, 4,6-Diaminopyrogallol-dihydrochlorid, 3,5-Diamino-4-hydroxybrenzcatechin-sulfat, aromatische Nitrile, Nitrogruppen-haltige Aminoverbindungen, wie 3-Amino-6-methylamino-2-nitro-pyridin, Pikraminsäure, 1-(2(3)-Nitro-4-amino)-phenylazo-2-hydroxy-7-trimethylammoniumnaphthalin-chlorid, 1-Hydroxy-2-amino-4,6-dinitro-benzol, 1-Amino-2-nitro-4-bis-(2-hydroxyethyl)-aminobenzol, 1-Amino-2-(2-hydroxyethyl)-amino-5-nitro-benzol (HC Yellow Nr. 5), 1-Amino-2-nitro-4-(2-hydroxyethyl)-aminobenzol (HC Red Nr. 7), 2-Chloro-5-nitro-N-hydroxyethyl-1,4-phenylendiamin, 1-(2-Hydroxyethyl)-amino-2-nitro-4-amino-benzol (HC Red Nr. 3), 4-Amino-3-nitrophenol, 4-Amino-2-nitrophenol, 6-Nitro-o-toluidin, 1-Amino-3-methyl-4-(2-hydroxyethyl)-amino-6-nitrobenzol (HC Violet Nr. 1), 1-Amino-2-nitro-4-(2,3-dihydroxypropyl)-amino-5-chlorobenzol (HC Red Nr. 10), 4-Amino-2-nitrodiphenyl-amino-2'-carbonsäure (2-(4-Amino-2-nitroanilino)-benzoesäure), 6-Nitro-2,5-diaminopyridin, 2-Amino-6-chloro-4-nitrophenol, 2-Amino-6-chloro-4-nitrophenol, 1-Amino-2-(3-nitrophenylazo)-7-phenylazo-8-naphthol-3,6-disulfonsäure, Dinatriumsalz (Acid blue Nr. 29), 1-Amino-2-(2-hydroxy-4-nitrophenylazo)-8-naphthol-3,6-disulfonsäure, Dinatriumsalz (Palatinchrome green), 1-Amino-2-(3-chlor-2-hydroxy-5-nitrophenylazo)-8-naphthol-3,6-disulfonsäure, Dinatriumsalz (Gallion), 4-Amino-4'-nitrostilben-2,2'-disulfonsäure, Dinatriumsalz, 2,4-Diamino-3',5'-dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2-nitrobenzylidenamino)-benzol, 2-[2-(Diethylamino)-ethylamino]-5-nitroanilin, 3-Amino-4-hydroxy-5-nitrobenzolsulfonsäure, 3-Amino-3'-nitrobiphenyl, 3-Amino-4-nitro-acenaphthen, 2-Amino-1-nitronaphthalin, 5-Amino-6-nitrobenzo-1,3-dioxol, 1,4-Bis-(4-aminophenyl)-1,4-diazacycloheptan, Bis-(5-amino-2-hydroxyphenyl)-methan, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 2-Amino-6-chlor-4-nitrophenol, 1-Amino-5-chlor-4-(2-hydroyethylamino)-2-nitrobenzol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 2-Amino-6-chlor-4-nitrophenol, 1-Amino-5-chlor-4-(2-hydroyethylamino)-2-nitrobenzol, aromatische Aniline bzw. Phenole mit einem weiteren aromatischen Rest wie 4,4'-Diaminostilben-dihydrochlorid, 4,4'-Diaminostilben-2,2'-disulfonsäure, Na-Salz, 4,4'-Diaminodiphenylmethan, -sulfid, -sulfoxid, -amin, 4,4'-Diaminodiphenylamin-2-sulfonsäure, 4,4'-Diaminobenzophenon, -diphenylether, 3,3',4,4'-Tetraaminodiphenyl-tetrahydrochlorid, 3,3',4,4'-Tetraamino-benzophenon, 1,3-Bis-(2,4-diaminophenoxy)-propan-tetrahydrochlorid, 1,8-Bis-(2,5-diaminophenoxy)-3,6-dioxaoctan-tetrahydrochlorid, 1,3-Bis-(4-aminophenylamino)-propan, -2-propanol, 1,3-Bis-[N-(4-aminophenyl)-2-hydroxyethylamino]-2-propanol, N,N-Bis-[2-(4-aminophenoxy)-ethyl]methylamintrihydrochlorid,
stickstoffhaltigen heterocyclischen Verbindungen ausgewählt aus der Gruppe, bestehend aus 2-, 3-, 4-Amino-, 2-Amino-3-hydroxy-, 2,6-Diamino-, 2,5-Diamino-, 2,3-Diamino-, 2-Dimethylamino-5-amino-, 3-Amino-2-methylamino-6-methoxy-, 2,3-Diamino-6-methoxy-, 3,5-Diamino-2,6-dimethoxy-, 2,4,5-Triamino-, 2,6-Dihydroxy-3,4-dimethylpyridin, 4,5,6-Triamino-, 2-Hydroxy-4,5,6-triamino-, 4-Hydroxy-2,5,6-triamino-, 2,4,5,6-Tetraamino-, 2-Methylamino-4,5,6-triamino-, 2,4-, 4,5-Diamino-, 2-Amino-4-methoxy-6-methyl-pyrimidin, 2,3,4-Trimethylpyrrol, 2,4-Dimethyl-3-ethyl-pyrrol, 3,5-Diaminopyrazol, -1,2,4-triazol, 3-Amino-, 3-Amino-5-hydroxypyrazol, 4,5-Diamino-1-(2-hydroxyethyl)-pyrazol, 2-, 3-, 8-Aminochinolin, 4-Amino-chinaldin, 2-, 6-Aminonicotinsäure, 5-Aminoisochinolin, 5-, 6-Aminoindazol, 5-, 7-Amino-benzimidazol, -benzothiazol, 2,5-Dihydroxy-4-morpholinoanilin sowie Indol- und Indolinderivaten, wie 4-, 5-, 6-, 7-Aminoindol, 4-, 5-, 6-, 7-Hydroxyindol, 5,6-Dihydroxyindol, 5,6-Dihydroxyindolin, 4-Hydroxyindolin und die in DE-U1-299 08 573 offenbarten Hydroxypyrimidine, sowie jeweils aus den mit vorzugsweise anorganischen Säuren gebildeten physiologisch verträglichen Salzen dieser Verbindungen,
aromatischen Hydroxyverbindungen ausgewählt aus der Gruppe, bestehend aus 2-, 4-, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2-Hydroxyethyl)-, 3,4-Methylendioxyphenol, 2,4-, 3,4-Dihydroxybenzoesäure, -phenylessigsäure, Gallussäure, 2,4,6-Trihydroxybenzoesäure, - acetophenon, 2-, 4-Chlorresorcin, 1-Naphthol, 1,5-, 2,3-, 2,7-Dihydroxynaphthalin, 6-Dimethylamino-4-hydroxy-2-naphthalinsulfonsäure, 3,6-Dihydroxy-2,7-naphthalinsulfonsäure,
Aminosäuren ausgewählt aus der Gruppe Arginin, Histidin, Tyrosin, Phenylalanin, DOPA (Dihydroxyphenylalanin), Ornithin, Lysin und Tryptophan,
Oligopeptid ausgewählt aus Glutathion oder die in den Hydrolysaten von Kollagen, Keratin, Casein, Elastin, Sojaprotein, Weizengluten oder Mandelprotein enthaltene Oligopeptide,
CH-aktiven Verbindungen ausgewählt aus der Gruppe, bestehend aus 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, Fischersche Base (1,3,3-Trimethyl-2-methylenindolin), 2,3-Dimethyl-benzothiazoliumiodid, 2,3-Dimethyl-benzothiazolium-p-toluolsulfonat, 1,2-Dimethyl-naphtho[1,2-d]thiazolium-p-toluolsulfonat, 1-Ethyl-2-methyl-naphtho[1,2-d]thiazolium-p-toluolsulfonat, Rhodanin, Rhodanin-3-essigsäure, 1-Ethyl-2-chinaldinium-iodid, 1,4-Dimethylchinolinium-iodid, 1-Methyl-2-chinaldinium-iodid, 1,4-Dimethylchinolinium-iodid, Barbitursäure, Thiobarbitursäure, 1,3-Dimethylthiobarbitursäure, 1,3-Diethylthiobarbitursäure, Diethylthiobarbitursäure, Oxindol, 3-Indoxylacetat, Cumaranon, 1-Methyl-3-phenyl-2-pyrazolinon, Indan-1,3-dion, Indan-1-on, 1-Dicyanmethylenindan.

6. Mittel nach Anspruch 5, **dadurch gekennzeichnet, dass** die weitere Verbindung ausgewählt ist aus der Gruppe bestehend aus N-(2-Hydroxyethyl)-N-ethyl-, 2-Chlor-p-phenylendiamin, N,N-Bis-(2-Hydroxyethyl)-p-phenylendiamin, 4-Aminophenol, p-Phenylendiamin, 2-(2,5-Diaminophenyl)-ethanol, 2,5-Diaminotoluol, 3,4-Methylendioxyanilin, 2-Amino-4-(2-hydroxyethylamino)-anisol, 2-(2,4-Diaminophenoxy)-ethanol, 3-Amino-2,4-dichlor-, 2-Methyl-5-amino-, 3-Methyl-4-amino-, 2-Methyl-5-(2-hydroxyethylamino)-, 2-Methyl-5-amino-4-chlor-, 6-Methyl-3-amino-2-chlor-, 2-Aminomethyl-4-aminophenol, 2-Diethylaminomethyl-4-aminophenol, 2-Dimethylaminomethyl-4-aminophenol, 2,6-Dichlor-4-aminophenol, 2-Hydroxymethyl-4-aminophenol, 3,4-Methylendioxyphenol, 3,4-Diaminobenzoesäure, 2,5-Diamino-, 2-Dimethylamino-5-amino-, 3-Amino-2-methylamino-6-methoxy-, 2,3-Diamino-6-methoxy-, 3,5-Diamino-2,6-dimethoxy-, 2,6-Dihydroxy-3,4-dimethylpyridin, 2-Hydroxy-4,5,6-triamino-, 4-Hydroxy-2,5,6-triamino-, 2,4,5,6-Tetraamino-, 2-Methylamino-4,5,6-triamino-pyrimidin, 3,5-Diaminopyrazol, 3-Amino-5-hydroxypyrazol, 4,5-Diamino-1-(2-hydroxyethyl)-pyrazol, 5,6-Dihydroxyindol und 5,6-Dihydroxyindolin, β-Alanin, L-Prolin, L-Lysin, DL-Tyrosin sowie jeweils aus den vorzugsweise mit anorganischen Säuren gebildeten physiologisch verträglichen Salzen dieser Verbindungen.

7. Mittel nach einem der Ansprüche 1 bis 6, **dadurch gekennzeichnet, dass** es Farbverstärker ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazidin oder deren beliebigen Gemischen enthält.

8. Mittel nach einem der Ansprüche 1 bis 7, **dadurch gekennzeichnet, dass** es direkt ziehende Farbstoffe aus der Gruppe der Nitrophenylendiamine, Nitroaminophenole, Anthrachinone oder Indophenole vorzugsweise in einer Menge von 0,01 bis 20 Gew.-%, bezogen auf das gesamte Färbemittel, enthält.

9. Mittel nach einem der Ansprüche 1 bis 8, **dadurch gekennzeichnet, dass** Ammonium- oder Metallsalze ausgewählt aus der Gruppe der Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, zugegeben werden.

10. Mittel nach einem der Ansprüche 1 bis 9, **dadurch gekennzeichnet, dass** es Oxidationsmittel, insbesondere H₂O₂, in einer Menge von 0,01 bis 6 Gew.-%, bezogen auf die Anwendungslösung, enthält.

11. Mittel nach einem der Ansprüche 1 bis 10, **dadurch gekennzeichnet, dass** es anionische, zwitterionische oder nichtionische Tenside enthält.

12. Verwendung von Chinoxalinderivaten mit der Formel Ia oder Ib oder Ic, 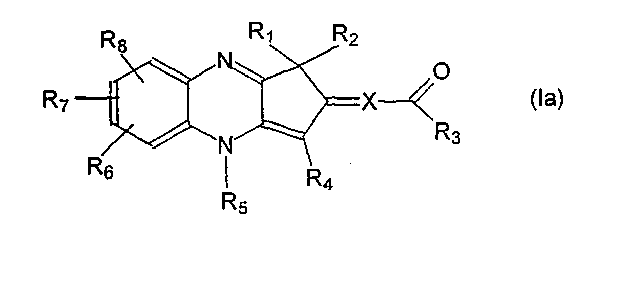 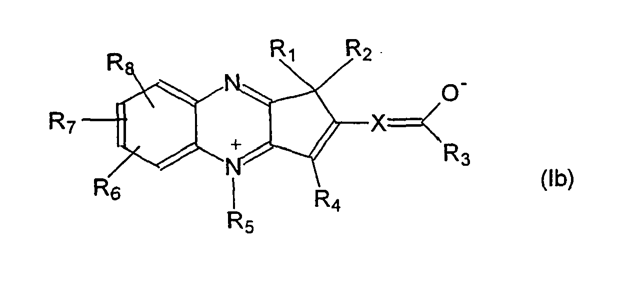 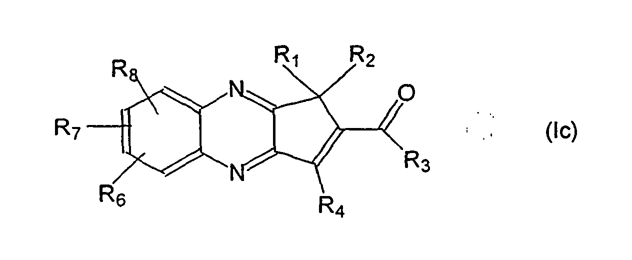 in der bedeuten
R¹, R², R³, R⁴ ein Wasserstoffatom oder eine C₁-C₄-Alkylgruppe,
R⁵ ein Wasserstoffatom, eine C₁-C₆-Alkyl- oder Aralkyl-, Aryl-, C₁-C₄-Alkenyl-, Hydroxyalkyl-, Carboxyalkylgruppe,
R⁶, R⁷, R⁸ ein Wasserstoff-, Halogenatom, eine C₁-C₄-Alkyl-, C₁-C₄-Alkoxy-, Nitro-, Amino-, Alkylamino-, Hydroxy-, Carboxy-, Sulfo-, Thiol-, oder C₁-C₄-Alkylthiogruppe,
wobei je zwei Reste auch gemeinsam einen Ring bilden können, wobei -X= bzw. =X-für -CH=, =CH- steht,
als eine färbende Komponente in Oxidationshaarfärbemitteln.

13. Verfahren zum Färben von keratinhaltigen Fasern, insbesondere menschlichen Haaren, worin ein Färbemittel, enthaltend
A) mindestens ein Chinoxalinderivat mit der Formel la oder Ib oder Ic 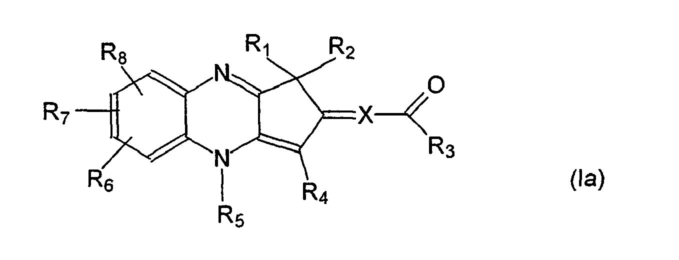 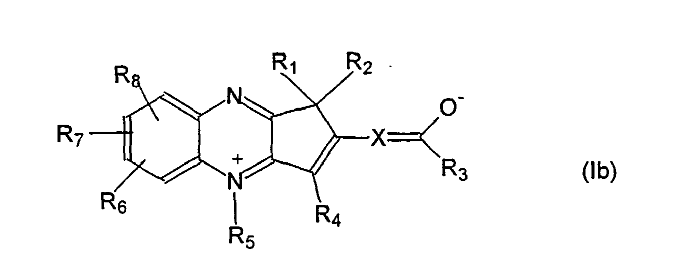 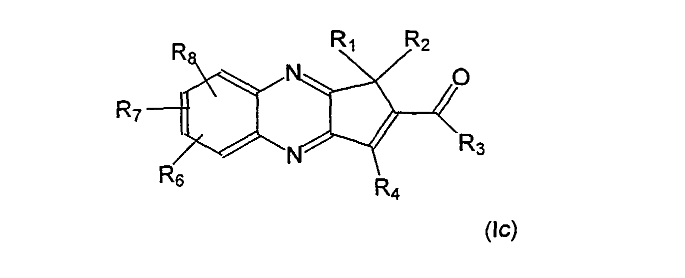 in der bedeuten
R¹, R², R³, R⁴ unabhängig voneinander ein Wasserstoffatom oder eine C₁-C₄₋Alkylgruppe,
R⁵ ein Wasserstoffatom, eine C₁-C₆-Alkyl- oder Aralkyl-, Aryl-, C₁-C₄-Alkenyl-, Hydroxyalkyl-, Carboxyalkylgruppe,
R⁶, R⁷, R⁸ unabhängig voneinander ein Wasserstoff-, Halogenatom, eine C₁-C₄₋Alkyl-, C₁-C₄-Alkoxy-, Nitro-, Amino-, Alkylamino-, Hydroxy-, Carboxy-, Sulfo-, Thiol-, oder C₁-C₄-Alkylthiogruppe, wobei je zwei Reste auch gemeinsam einen Ring bilden können,
wobei -X= bzw. =X- für -CH=, =CH- steht,
und
B) mindestens eine Verbindung mit primärer oder sekundärer Aminogruppe oder Hydroxygruppe, ausgewählt aus primären oder sekundären aromatischen Aminen, stickstoffhaltigen heterocyclischen Verbindungen und aromatischen Hydroxyverbindungen, Aminosäuren, aus 2 bis 9 Aminosäuren aufgebauten Oligopeptiden und/oder mindestens eine CH-aktive Verbindung,
sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

## Claims

1. Agent for dyeing fibres containing keratin, in particular human hair, comprising, as dyeing component, at least one quinoxaline derivative with the formula Ia or Ib or Ic, 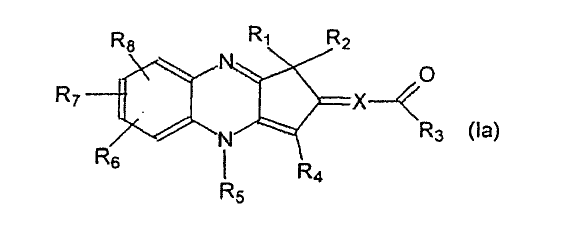 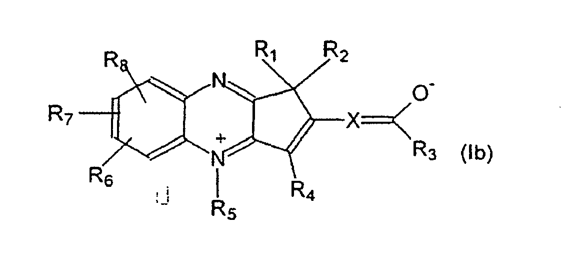 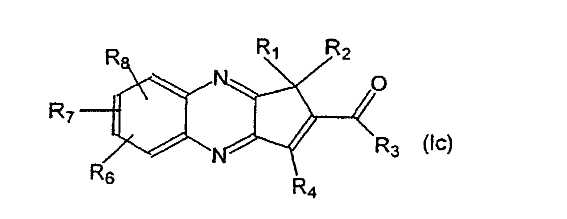 in which
R¹, R², R³, R⁴, independently of one another, are a hydrogen atom or a C₁-C₄-alkyl group,
R⁵ is a hydrogen atom, a C₁-C₆-alkyl group or aralkyl group, aryl group, C₁-C₄-alkenyl group, hydroxyalkyl group, carboxyalkyl group,
R⁶, R⁷, R⁸, independently of one another, are a hydrogen atom, halogen atom, a C₁-C₄-alkyl group, C₁-C₄-alkoxy group, nitro group, amino group, alkylamino group, hydroxy group, carboxy group, sulpho group, thiol group, or C₁-C₄-alkylthio group, where two radicals in each case can also together form a ring,
where -X= or =X- is -CH=, =CH-.

2. Agent according to Claim 1, **characterized in that** the compounds with the formula Ia or Ib or Ic used are 1,1,3-trimethyl-1H-cyclopenta[b]quinoxaline-2-carbaldehyde, 6 (7) -chloro-, 6 (7) -fluoro-, 6(7)-bromo-, 6(7)-methyl-, 6(7)-nitro-, 6,7-dichloro-, 6,7-methylenedioxy-1,1,3-trimethyl-1H-cyclopenta-[b]quinoxaline-2-carbaldehyde, 1,1,3,6(7)-tetramethyl-, 1,1,3,6,7-pentamethyl-1H-cyclopenta[b]-quinoxaline-2-carbaldehyde, 1,1,3-trimethyl-1H-benzo[g]cyclopenta[b]quinoxaline-2-carbaldehyde, 2,4-dihydro-1,1,3,4-tetramethyl-1H-cyclopenta[b]-quinoxalin-2-ylideneacetaldehyde, 2,4-dihydro-4-phenyl-1,1,3-trimethyl-1H-cyclopenta[b]quinoxalin-2-ylideneacetaldehyde, 6,7-dichloro-2,4-dihydro-1,1,3,4-tetramethyl-1H-cyclopenta[b]quinoxalin-2-ylideneacetaldehyde, 7-chloro-2,4-dihydro-4-(2-propyl)-1,1,3-trimethyl-1H-cyclopenta[b]-quinoxalin-2-ylideneacetaldehyde, 2,4-dihydro-6,7-methylenedioxy-1,1,3,4-tetramethyl-1H-cyclopenta-[b]quinoxalin-2-ylideneacetaldehyde, and any mixtures thereof.

3. Agent according to one of Claims 1 to 2, **characterized in that** the quinoxaline derivatives of the formula Ia or Ib or Ic are present in an amount of from 0.03 to 65 mmol, in particular from 1 to 40 mmol, based on 100 g of the total colorant.

4. Agent according to one of Claims 1 to 3, **characterized in that** it additionally comprises at least one compound with a primary or secondary amino group or hydroxy group chosen from primary or secondary aromatic amines, nitrogen-containing heterocyclic compounds and aromatic hydroxy compounds, amino acids, oligopeptides constructed from 2 to 9 amino acids and/or at least one CH-active compound.

5. Agent according to Claim 4, **characterized in that** the further compound is chosen from primary or secondary amines from the group consisting of N-(2-hydroxyethyl)-N-ethyl-, N-(2-methoxyethyl-), 2,3-, 2,4-, 2,5-dichloro-p-phenylenediamine, 2-chloro-p-phenylenediamine, N,N-bis(2-hydroxyethyl)-p-phenylenediamine, 2,5-dihydroxy-4-morpholinoaniline dihydrobromide, 2-, 3-, 4-aminophenol, o-, m-, p-phenylenediamine, 2,4-diaminophenoxyethanol, 2-(2,5-diaminophenyl)-ethanol, 2,5-diaminotoluene, -phenol, -phenetol, 4-methylamino-, 3-amino-4-(2'-hydroxyethyloxy)-, 3,4-methylenediamino-, 3,4-methylenedioxyaniline, 3-amino-2,4-dichloro-, 4-methylamino-, 2-methyl-5-amino-, 3-methyl-4-amino-, 2-methyl-5-(2-hydroxyethylamino)-, 2-methyl-5-amino-4-chloro-, 6-methyl-3-amino-2-chloro-, 5-(2-hydroxyethylamino)-4-methoxy-2-methyl-, 4-amino-2-aminomethylphenol, 2-hydroxymethyl-4-aminophenol, 2-diethylaminomethyl-4-aminophenol, 2-dimethylaminomethyl-4-aminophenol, 2,6-dichloro-4-aminophenol, 1,3-diamino-2,4-dimethoxybenzene, 2-, 3-, 4-aminobenzoic acid, -phenylacetic acid, 2,3-, 2,4-, 2,5-, 3,4-, 3,5-diaminobenzoic acid, 4-, 5-aminosalicylic acid, 3-amino-4-hydroxy-, 4-amino-3-hydroxybenzoic acid, 2-, 3-, 4-aminobenzenesulphonic acid, 3-amino-4-hydroxybenzenesulphonic acid, 4-amino-3-hydroxynaphthalenesulphonic acid, 6-amino-7-hydroxynaphthalene-2-sulphonic acid, 7-amino-4-hydroxynaphthalene-2-sulphonic acid, 4-amino-5-hydroxynaphthalene-2,7-disulphonic acid, 3-amino-2-naphthoic acid, 3-aminophthalic acid, 5-aminoisophthalic acid, 1,3,5-, 1,2,4-triaminobenzene, 1,2,4,5-tetraaminobenzene tetrahydrochloride, 2,4,5-triaminophenol trihydrochloride, pentaaminobenzene pentahydrochloride, hexaaminobenzene hexahydrochloride, 2,4,6-triaminoresorcinol trihydrochloride, 4,5-diaminopyrocatechin sulphate, 4,6-diaminopyrogallol dihydrochloride, 3,5-diamino-4-hydroxypyrocatechin sulphate, aromatic nitriles, amino compounds containing nitro groups, such as 3-amino-6-methylamino-2-nitropyridine, picramic acid, 1-(2(3)-nitro-4-amino)phenylazo-2-hydroxy-7-trimethylammonium naphthalene chloride, 1-hydroxy-2-amino-4,6-dinitrobenzene, 1-amino-2-nitro-4-bis(2-hydroxyethyl)aminobenzene, 1-amino-2-(2-hydroxyethyl)-amino-5-nitrobenzene (HC Yellow No. 5), 1-amino-2-nitro-4-(2-hydroxyethyl)aminobenzene (HC Red No. 7), 2-chloro-5-nitro-N-hydroxyethyl-1,4-phenylenediamine, 1-(2-hydroxyethyl)amino-2-nitro-4-aminobenzene (HC Red No. 3), 4-amino-3-nitrophenol, 4-amino-2-nitrophenol, 6-nitro-o-toluidine, 1-amino-3-methyl-4-(2-hydroxyethyl)-amino-6-nitrobenzene (HC Violet No. 1), 1-amino-2-nitro-4-(2,3-dihydroxypropyl)amino-5-chlorobenzene (HC Red No. 10), 4-amino-2-nitrodiphenylamino-2'-carboxylic acid (2-(4-amino-2-nitroanilino)benzoic acid), 6-nitro-2,5-diaminopyridine, 2-amino-6-chloro-4-nitrophenol, 2-amino-6-chloro-4-nitrophenol, 1-amino-2-(3-nitrophenylazo)-7-phenylazo-8-naphthol-3,6-disulphonic acid, disodium salt (Acid blue No. 29), 1-amino-2-(2-hydroxy-4-nitrophenylazo)-8-naphthol-3,6-disulphonic acid, disodium salt (Palatine chrome green), 1-amino-2-(3-chloro-2-hydroxy-5-nitrophenylazo)-8-naphthol-3,6-disulphonic acid, disodium salt (Gallion), 4-amino-4'-nitrostilbene-2,2'-disulphonic acid, disodium salt, 2,4-diamino-3',5'-dinitro-2'-hydroxy-5-methylazobenzene (Mordant brown 4), 4'-amino-4-nitrodiphenylamine-2-sulphonic acid, 4'-amino-3'-nitrobenzophenone-2-carboxylic acid, 1-amino-4-nitro-2-(2-nitrobenzylideneamino)-benzene, 2-[2-(diethylamino)ethylamino]-5-nitroaniline, 3-amino-4-hydroxy-5-nitrobenzenesulphonic acid, 3-amino-3'-nitrobiphenyl, 3-amino-4-nitroacenaphthene, 2-amino-1-nitronaphthalene, 5-amino-6-nitrobenzo-1,3-dioxol, 1,4-bis(4-aminophenyl)-1,4-diazacycloheptane, bis(5-amino-2-hydroxyphenyl)methane, anilines, in particular anilines containing nitro groups, such as 4-nitroaniline, 2-nitroaniline, 1,4-diamino-2-nitrobenzene, 1,2-diamino-4-nitrobenzene, 1-amino-2-methyl-6-nitrobenzene, 4-nitro-1,3-phenylenediamine, 2-nitro-4-amino-1-(2-hydroxyethylamino)benzene, 2-nitro-1-amino-4-[bis(2-hydroxyethyl)amino]-benzene, 4-amino-2-nitrodiphenylamine-2'-carboxylic acid, 2-amino-6-chloro-4-nitrophenol, 1-amino-5-chloro-4-(2-hydroxyethylamino)-2-nitrobenzene, anilines, in particular anilines containing nitro groups, such as 4-nitroaniline, 2-nitroaniline, 1,4-diamino-2-nitrobenzene, 1,2-diamino-4-nitrobenzene, 1-amino-2-methyl-6-nitrobenzene, 4-nitro-1,3-phenylenediamine, 2-nitro-4-amino-1-(2-hydroxyethylamino)benzene, 2-nitro-1-amino-4-[bis(2-hydroxyethyl)amino]-benzene, 4-amino-2-nitrodiphenylamine-2'-carboxylic acid, 2-amino-6-chloro-4-nitrophenol, 1-amino-5-chloro-4-(2-hydroxyethylamino)-2-nitrobenzene, aromatic anilines and phenols with a further aromatic radical, such as 4,4'-diaminostilbene dihydrochloride, 4,4'-diaminostilbene-2,2'-disulphonic acid, Na salt, 4,4'-diaminodiphenylmethane, sulphide, sulphoxide, -amine, 4,4'-diaminodiphenylamine-2-sulphonic acid, 4,4'-diaminobenzophenone, -diphenyl ether, 3,3',4,4'-tetraaminodiphenyl tetrahydrochloride, 3,3'-4,4'-tetraaminobenzophenone, 1,3-bis(2,4-diaminophenoxy)propane tetrahydrochloride, 1,8-bis-(2,5-diaminophenoxy)-3,6-dioxaoctane tetrahydrochloride, 1,3-bis(4-aminophenylamino)propane, -2-propanol, 1,3-bis[N-(4-aminophenyl)-2-hydroxyethylamino]-2-propanol, N,N-bis[2-(4-aminophenoxy)ethyl]methylamine trihydrochloride, nitrogen-containing heterocyclic compounds chosen from the group consisting of 2-, 3-, 4-amino-, 2-amino-3-hydroxy-, 2,6-diamino-, 2,5-diamino-, 2,3-diamino-, 2-dimethylamino-5-amino-, 3-amino-2-methylamino-6-methoxy-, 2,3-diamino-6-methoxy-, 3,5-diamino-2,6-dimethoxy-, 2,4,5-triamino-, 2,6-dihydroxy-3,4-dimethylpyridine, 4,5,6-triamino-, 2-hydroxy-4,5,6-triamino-, 4-hydroxy-2,5,6-triamino-, 2,4,5,6-tetraamino-, 2-methylamino-4,5,6-triamino-, 2,4-, 4,5-diamino-, 2-amino-4-methoxy-6-methylpyrimidine, 2,3,4-trimethylpyrrole, 2,4-dimethyl-3-ethylpyrrole, 3,5-diaminopyrazole, -1,2,4-triazole, 3-amino-, 3-amino-5-hydroxypyrazole, 4,5-diamino-1-(2-hydroxyethyl)pyrazole, 2-, 3-, 8-aminoquinoline, 4-aminoquinaldine, 2-, 6-aminonicotinic acid, 5-aminoisoquinoline, 5-, 6-aminoindazole, 5-, 7-aminobenzimidazole, -benzothiazole, 2,5-dihydroxy-4-morpholinoaniline, and indole and indoline derivatives, such as 4-, 5-, 6-, 7-aminoindole, 4-, 5-, 6-, 7-hydroxyindole, 5,6-dihydroxyindole, 5,6-dihydroxyindoline, 4-hydroxyindoline and the hydroxypyrimidines disclosed in DE-U1-299 08 573, and in each case from physiologically compatible salts of these compounds formed with preferably inorganic acids, aromatic hydroxy compounds chosen from the group consisting of 2-, 4-, 5-methylresorcinol, 2,5-dimethylresorcinol, resorcinol, 3-methoxyphenol, pyrocatechin, hydroquinone, pyrogallol, phloroglucine, hydroxyhydroquinone, 2-, 3-, 4-methoxy-, 3-dimethylamino-, 2-(2-hydroxyethyl)-, 3,4-methylenedioxyphenol, 2,4-, 3,4-dihydroxybenzoic acid, -phenylacetic acid, gallic acid, 2,4,6-trihydroxybenzoic acid, -acetophenone, 2-, 4-chlororesorcinol, 1-naphthol, 1,5-, 2,3-, 2,7-dihydroxynaphthalene, 6-dimethylamino-4-hydroxy-2-naphthalenesulphonic acid, 3,6-dihydroxy-2,7-naphthalenesulphonic acid,
amino acids chosen from the group consisting of arginine, histidine, tyrosine, phenylalanine, DOPA (dihydroxyphenylalanine), ornithine, lysine and tryptophan,
oligopeptide chosen from glutathione or the oligopeptides present in the hydrolysates of collagen, keratin, casein, elastin, soya protein, wheat gluten or almond protein,
CH-active compounds chosen from the group consisting of 1,2,3,3-tetramethyl-3H-indolium-iodide, 1,2,3,3-tetramethyl-3H-indolium p-toluenesulphonate, 1,2,3,3-tetramethyl-3H-indolium methanesulphonate, Fischer's base (1,3,3-trimethyl-2-methyleneindoline), 2,3-dimethylbenzothiazolium iodide, 2,3-dimethylbenzothiazolium p-toluenesulphonate, 1,2-dimethylnaphtho[1,2-d]-thiazolium p-toluenesulphonate, 1-ethyl-2-methyl-naphtho[1,2-d]thiazolium p-toluenesulphonate, rhodanine, rhodanine-3-acetic acid, 1-ethyl-2-quinaldinium iodide, 1,4-dimethylquinolinium iodide, 1-methyl-2-quinaldinium iodide, 1,4-dimethylquinolinium iodide, barbituric acid, thiobarbituric acid, 1,3-dimethylthiobarbituric acid, 1,3-diethylthiobarbituric acid, diethylthiobarbituric acid, oxindole, 3-indoxyl acetate, coumaranone, 1-methyl-3-phenyl-2-pyrazolinone, indane-1,3-dione, indan-1-one, 1-dicyanomethylene-indane.

6. Agent according to Claim 5, **characterized in that** the further compound is chosen from the group consisting of N-(2-hydroxyethyl)-N-ethyl-, 2-chloro-p-phenylenediamine, N,N-bis(2-hydroxyethyl)-p-phenylenediamine, 4-aminophenol, p-phenylenediamine, 2-(2,5-diaminophenyl)ethanol, 2,5-diaminotoluene, 3,4-methylenedioxyaniline, 2-amino-4-(2-hydroxyethylamino)anisole, 2-(2,4-diaminophenoxy)ethanol, 3-amino-2,4-dichloro-, 2-methyl-5-amino-, 3-methyl-4-amino-, 2-methyl-5-(2-hydroxyethylamino)-, 2-methyl-5-amino-4-chloro-, 6-methyl-3-amino-2-chloro-, 2-aminomethyl-4-aminophenol, 2-diethylaminomethyl-4-aminophenol, 2-dimethylaminomethyl-4-aminophenol, 2,6-dichloro-4-aminophenol, 2-hydroxymethyl-4-aminophenol, 3,4-methylenedioxyphenol, 3,4-diaminobenzoic acid, 2,5-diamino-, 2-dimethylamino-5-amino-, 3-amino-2-methylamino-6-methoxy-, 2,3-diamino-6-methoxy-, 3,5-diamino-2,6-di-methoxy-, 2,6-dihydroxy-3,4-dimethylpyridine, 2-hydroxy-4,5,6-triamino-, 4-hydroxy-2,5,6-triamino-, 2,4,5,6-tetraamino-, 2-methylamino-4,5,6-triaminopyrimidine, 3,5-diaminopyrazole, 3-amino-5-hydroxypyrazole, 4,5-diamino-1-(2-hydroxyethyl)-pyrazole, 5,6-dihydroxyindole and 5,6-dihydroxyindoline, β-alanine, L-proline, L-lysine, DL-tyrosine, and in each case from the physiologically compatible salts of the compounds formed preferably with inorganic acids.

7. Agent according to one of Claims 1 to 6, **characterized in that** it comprises colour enhancers chosen from the group consisting of piperidine, piperidine-2-carboxylic acid, piperidine-3-carboxylic acid, piperidine-4-carboxylic acid, pyridine, 2-hydroxypyridine, 3-hydroxypyridine, 4-hydroxypyridine, imidazole, 1-methylimidazole, histidine, pyrrolidine, proline, pyrrolidone, pyrrolidone-5-carboxylic acid, pyrazole, 1,2,4-triazole, piperazidine or any mixtures thereof.

8. Agent according to one of Claims 1 to 7, **characterized in that** it comprises direct dyes from the group of nitrophenylenediamines, nitroaminophenols, anthraquinones or indophenols, preferably in an amount of from 0.01 to 20% by weight, based on the total colorant.

9. Agent according to one of Claims 1 to 8, **characterized in that** ammonium or metal salts chosen from the group of formates, carbonates, halides, sulphates, butyrates, valerates, capronates, acetates, lactates, glycolates, tartrates, citrates, gluconates, propionates, phosphates and phosphonates of alkali metals, such as potassium, sodium or lithium, alkaline earth metals, such as magnesium, calcium, strontium or barium, or of aluminium, manganese, iron, cobalt, copper or zinc, are added.

10. Agent according to one of Claims 1 to 9, **characterized in that** it comprises oxidizing agents, in particular H₂O₂, in an amount of from 0.01 to 6% by weight, based on the application solution.

11. Agent according to one of Claims 1 to 10, **characterized in that** it comprises anionic, zwitterionic or nonionic surfactants.

12. Use of quinoxaline derivatives with the formula Ia or Ib or Ic, 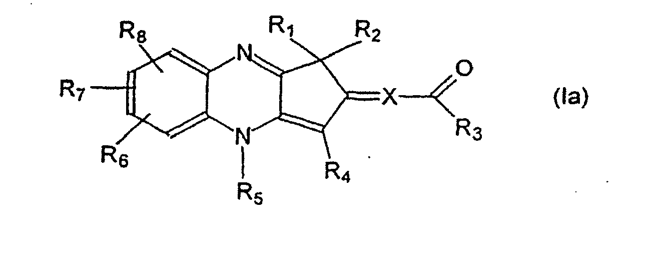 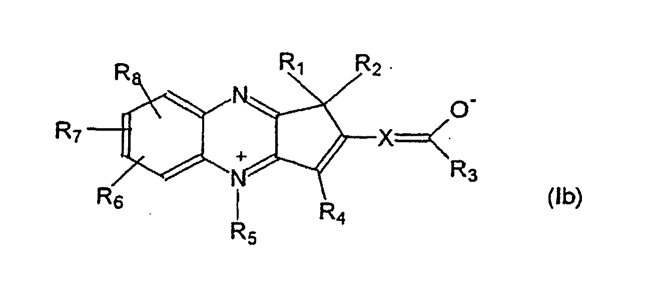 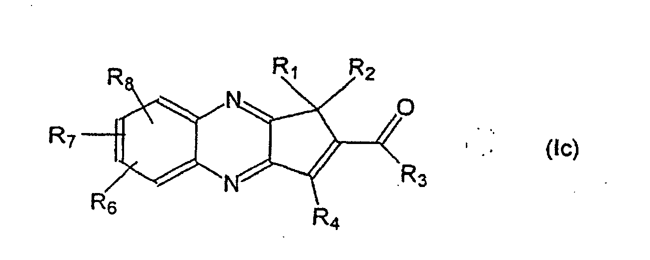 in which
R¹, R², R³, R⁴, are a hydrogen atom or a C₁-C₄-alkyl group,
R⁵ is a hydrogen atom, a C₁-C₆-alkyl group or aralkyl group, aryl group, C₁-C₄-alkenyl group, hydroxyalkyl group, carboxyalkyl group,
R⁶, R⁷, R⁸, are a hydrogen atom, halogen atom, a C₁₋C₄-alkyl group, C₁-C₄-alkoxy group, nitro group, amino group, alkylamino group, hydroxy group, carboxy group, sulpho group, thiol group, or C₁-C₄₋alkylthio group, where in each case two radicals may also together form a ring, where -X= or =X- is -CH=, =CH-,
as a dyeing component in oxidation hair colorants.

13. Method for dyeing fibres containing keratin, in particular human hair, in which a colorant comprising
a) at least one quinoxaline derivative of the formula Ia or Ib or Ic 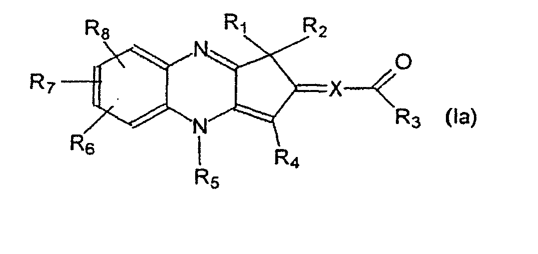 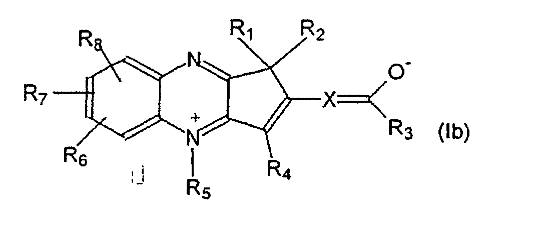 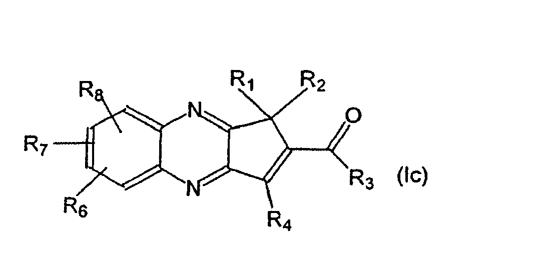 in which
R¹, R², R³, R⁴, independently of one another, are a hydrogen atom or a C₁-C₄-alkyl group,
R⁵ is a hydrogen atom, a C₁-C₆-alkyl group or aralkyl group, aryl group, C₁-C₄-alkenyl group, hydroxyalkyl group, carboxyalkyl group,
R⁶, R⁷, R⁸, independently of one another, are a hydrogen atom, halogen atom, a C₁-C₄-alkyl group, C₁-C₄-alkoxy group, nitro group, amino group, alkylamino group, hydroxy group, carboxy group, sulpho group, thiol group, or C₁-C₄₋alkylthio group, where two radicals in each case can also together form a ring,
where -X= or =X- is -CH=, =CH-,
and
B) at least one compound with a primary or secondary amino group or hydroxy group chosen from primary or secondary aromatic amines, nitrogen-containing heterocyclic compounds and aromatic hydroxy compounds, amino acids, oligopeptides constructed from 2 to 9 amino acids and/or at least one CH-active compound,
and customary cosmetic ingredients is applied to the fibres containing keratin, left on the fibres for a certain amount of time, usually about 30 minutes, and then rinsed out again or washed out with a shampoo.

## Revendications

1. Agent pour teindre des fibres kératiniques, en particulier des cheveux humains, contenant en tant que composant colorant au moins un dérivé quinoxaline de formule la ou Ib ou Ic, 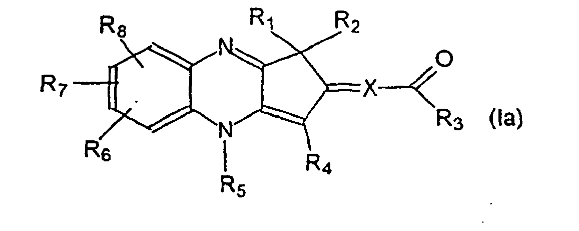 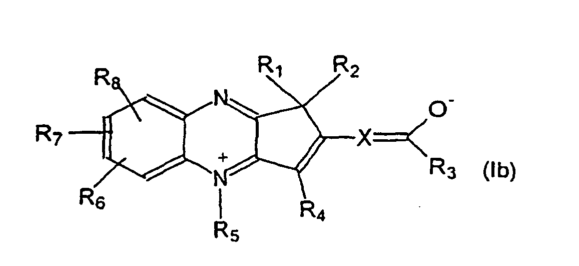 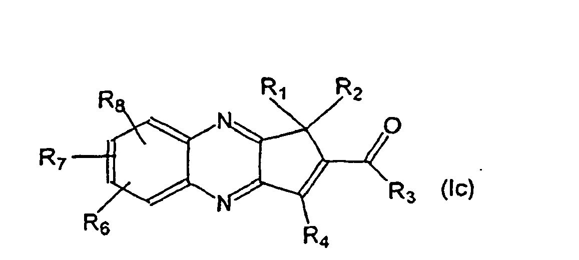 dans laquelle
R¹, R², R³, R⁴ représentent indépendamment l'un de l'autre, un atome d'hydrogène ou un groupe alkyle en C₁ à C₄,
R⁵ représente un atome d'hydrogène, un groupe alkyle en C₁ à C₆, ou un groupe aralkyle, aryle, alcényle en C₁ à C₄, hydroxyalkyle, carboxyalkyle,
R⁶, R⁷, R⁸ représentent indépendamment l'un de l'autre, un atome d'hydrogène, un atome d'halogène, un groupe alkyle en C₁ à C₄, alcoxy en C₁ à C₄, nitro, amino, alkylamino, hydroxy, carboxy, sulfo, thiol, ou alkylthio en C₁ à C₄, chacun des deux radicaux pouvant former également conjointement un cycle,
-X= où =X- représente -CH=, =CH-.

2. Agent selon la revendication 1, **caractérisé en ce que,** le 1,1,3-triméthyl-1H-cyclopenta[b]quinoxaline-2-carbaldéhyde, le 6(7)-chloro-, 6(7)-fluoro-, 6(7)-bromo-, 6(7)-méthyl-, 6(7)-nitro, 6,7-dichloro-, 6,7-méthylène-dioxy-1,1,3-triméthyl-1H-cyclopenta[b]quinoxaline-2-carbaldéhyde, le 1,1,3,6(7)-tétraméthyl-, 1,1,3,6,7-pentaméthyl-1H-cyclopenta[b]quinoxaline-2-carbaldéhyde, le 1,1,3-triméthyl-1 H-benzo[g]cyclopenta[b]quinoxaline-2-carbaldéhyde, le 2,4-dihydro-1,1,3,4-tétraméthyl-1 H-cyclopenta[b]quinoxalin-2-ylidène-acétaldéhyde, le 2,4-dihydro-4-phényl-1,1,3-triméthyl-1 H-cyclopenta[b]quinoxalin-2-ylidène-acétaldéhyde, le 6,7-dichloro-2,4-dihydro-1,1,3,4-tétraméthyl-1H-cyclopenta[b]quinoxalin-2-ylidène-acétaldéhyde, le 7-chloro-2,4-dihydro-4-(2-propyl)-1,1,3-triméthyl-1H-cyclopenta[b]quinoxalin-2-ylidène-acétaldéhyde, le 2,4-dihydro-6,7-méthylènedioxy-1,1,3,4-tétraméthyl-1H-cyclopenta[b]quinoxalin-2-ylidène-acétaldéhyde, ainsi que leurs mélanges quelconques, sont mis en oeuvre en tant que composé de formule Ia ou Ib ou Ic.

3. Agent selon l'une quelconque des revendications 1 à 2, **caractérisé en ce que** les dérivés quinoxaline de formule la ou Ib ou Ic sont contenus en une quantité de 0,03 à 65 mmoles, en particulier de 1 à 40 mmoles, par rapport à 100 g de la teinture totale.

4. Agent selon l'une quelconque des revendications 1 à 3, **caractérisé en ce qu'**il contient en plus au moins un composé ayant un groupe amino primaire ou secondaire ou un groupe hydroxy, choisi parmi les amines aromatiques primaires ou secondaires, les composés hétérocycliques azotés, et les composés hydroxyaromatiques, les acides aminés, les oligopeptides construits à partir de 2 à 9 acides aminés et/ou au moins un composé à groupe CH actif.

5. Agent selon la revendication 4, **caractérisé en ce que** le composé supplémentaire est choisi parmi
les amines primaires ou secondaires issues du groupe formé par la N-(2-hydroxyéthyl)-N-éthyl-, N-(2-méthoxyéthyl-), 2,3-, 2,4-, 2,5-dichloro-p-phénylène-diamine, la 2-chloro-p-phénylènediamine, la N,N-bis-(2-hydroxyéthyl)-p-phénylènediamine, le dibromhydrate de 2,5-dihydroxy-4-morpholinoaniline, le 2-, 3-, 4-aminophénol, la o-, m-, p-phénylènediamine, le 2,4-diaminophénoxyéthanol, le 2-(2,5-diaminophényl)-éthanol, le 2,5-diaminotoluène, 2,5-diaminophénol, 2,5-diaminophénéthol, la 4-méthylamino-, 3-amino-4-(2'-hydroxyéthyloxy)-, 3,4-méthylènediamino-, 3,4-méthylène-dioxyaniline, le 3-amino-2,4-dichloro-, 4-méthylamino-, 2-méthyl-5-amino-, 3-méthyl-4-amino-, 2-méthyl-5-(2-hydroxyéthylamino)-, 2-méthyl-5-amino-4-chloro-, 6-méthyl-3-amino-2-chloro-, 5-(2-hydroxyéthylamino)-4-méthoxy-2-méthyl-, 4-amino-2-aminométhyl-phénol, le 2-hydroxyméthyl-4-aminophénol, le 2-diéthylaminométhyl-4-aminophénol, le 2-diméthylaminométhyl-4-aminophénol, le 2,6-dichloro-4-aminophénol, le 1,3-diamino-2,4-diméthoxybenzène, l'acide 2-, 3-, 4-aminobenzoïque, l'acide 2-, 3-, 4-amino-phénylacétique, l'acide 2,3-, 2,4-, 2,5-, 3,4-, 3,5-diaminobenzoïque, l'acide 4-, 5-aminosalicylique, l'acide 3-amino-4-hydroxy-, 4-amino-3-hydroxybenzoïque, l'acide 2-, 3-, 4-aminobenzènesulfonique, l'acide 3-amino-4-hydroxybenzènesulfonique, l'acide 4-amino-3-hydroxynaphtalène-sulfonique, l'acide 6-amino-7-hydroxy-naphtalène-2-sulfonique, l'acide 7-amino-4-hydroxynaphtalène-2-sulfonique, l'acide 4-amino-5-hydroxynaphtalène-2,7-disulfonique, l'acide 3-amino-2-naphtoïque, l'acide 3-aminophtalique, l'acide 5-aminoisophtalique, le 1,3,5-, 1,2,4-triaminobenzène, le tétrachlorhydrate de 1,2,4,5-tétraaminobenzène, le trichlorhydrate de 2,4,5-triaminophénol, le pentachlorhydrate de pentaaminobenzène, l'hexachlorhydrate de hexaaminobenzène, le trichlorhydrate de 2,4,6-triaminorésorcine, le sulfate de 4,5-diaminocatéchol, le dichlorhydrate de 4,6-diaminopyrogallol, le sulfate de 3,5-diamino-4-hydroxycatéchol, les nitriles aromatiques, les composés amino contenant des groupes nitro, comme la 3-amino-6-méthylamino-2-nitro-pyridine, l'acide picramique, le chlorure de 1-(2(3)-nitro-4-amino)-phénylazo-2-hydroxy-7-triméthylammonium-naphtalène, le 1-hydroxy-2-amino-4,6-dinitro-benzène, le 1-amino-2-nitro-4-bis-(2-hydroxyéthyl)-aminobenzène, le 1-amino-2-(2-hydroxy-éthyl)-amino-5-nitro-benzène (HC Yellow N° 5), le 1-amino-2-nitro-4-(2-hydroxyéthyl)-aminobenzène (HC Red N° 7), la 2-chloro-5-nitro-N-hydroxyéthyl-1,4-phénylènediamine, le 1-(2-hydroxyéthyl)-amino-2-nitro-4-amino-benzène (HC Red N° 3), le 4-amino-3-nitrophénol, le 4-amino-2-nitrophénol, la 6-nitro-o-toluidine, le 1-amino-3-méthyl-4-(2-hydroxyéthyl)-amino-6-nitrobenzène (HC Violet N° 1), le 1-amino-2-nitro-4-(2,3-dihydroxypropyl)-amino-5-chlorobenzène (HC Red N° 10), l'acide 4-amino-2-nitrodiphényl-amino-2'-carboxylique, (l'acide 2-(4-amino-2-nitroanilino)-benzoïque), la 6-nitro-2,5-diaminopyridine, le 2-amino-6-chloro-4-nitrophénol, 2-amino-6-chloro-4-nitrophénol, l'acide 1-amino-2-(3-nitrophénylazo)-7-phénylazo-8-naphtol-3,6-disulfonique, sel disodique (Acid blue N° 29), l'acide 1-amino-2-(2-hydroxy-4-nitrophénylazo)-8-naphtol-3,6-disulfonique, sel disodique (Palatinchrome green), l'acide 1-amino-2-(3-chloro-2-hydroxy-5-nitrophénylazo)-8-naphtol-3,6-disulfonique, sel disodique (Gallion), l'acide 4-amino-4'-nitrostilbène-2,2'-disulfonique, sel disodique, le 2,4-diamino-3',5'-dinitro-2'-hydroxy-5-méthyl-azobenzène (Mordant brown 4), l'acide 4'-amino-4-nitrodiphénylamine-2-sulfonique, l'acide 4'-amino-3'-nitrobenzo-phénone-2-carboxylique, le 1-amino-4-nitro-2-(2-nitrobenzylidène-amino)-benzène, la 2-[2-(diéthylamino)-éthylamino]-5-nitroaniline, l'acide 3-amino-4-hydroxy-5-nitrobenzènesulfonique, le 3-amino-3'-nitrobiphényle, le 3-amino-4-nitro-acénaphtène, le 2-amino-1-nitronaphtalène, le 5-amino-6-nitrobenzo-1,3-dioxol, le 1,4-bis-(4-aminophényl)-1,4-diazacycloheptane, le bis-(5-amino-2-hydroxyphényl)-méthane, les anilines, en particulier les anilines contenant des groupes nitro, comme la 4-nitroaniline, la 2-nitroaniline, le 1,4-diamino-2-nitrobenzène, le 1,2-diamino-4-nitrobenzène, le 1-amino-2-méthyl-6-nitrobenzène, la 4-nitro-1,3-phénylènediamine, le 2-nitro-4-amino-1-(2-hydroxy-éthylamino)-benzène, le 2-nitro-1-amino-4-[bis-(2-hydroxyéthyl)-amino]-benzène, l'acide 4-amino-2-nitrodiphénylamin-2'-carboxylique, le 2-amino-6-chloro-4-nitrophénol, le 1-amino-5-chloro-4-(2-hydroxyéthylamino)-2-nitro-benzène, les anilines, en particulier les anilines contenant des groupes nitro, comme la 4-nitroaniline, la 2-nitroaniline, le 1,4-diamino-2-nitrobenzène, le 1,2-diamino-4-nitrobenzène, le 1-amino-2-méthyl-6-nitrobenzène, la 4-nitro-1,3-phénylènediamine, le 2-nitro-4-amino-1-(2-hydroxyéthylamino)-benzène, le 2-nitro-1-amino-4-[bis-(2-hydroxyéthyl)-amino]-benzène, l'acide 4-amino-2-nitrodiphénylamine-2'-carboxylique, le 2-amino-6-chloro-4-nitrophénol, le 1-amino-5-chloro-4-(2-hydroyéthylamino)-2-nitrobenzène, les anilines aromatiques ou les phénols avec un radical aromatique supplémentaire comme le dichlorhydrate de 4,4'-diaminostilbène, l'acide 4,4'-diaminostilbène-2,2'-disulfonique, sel de Na, le 4,4'-diaminodiphénylméthane, son sulfure, son sulfoxyde, son amine, l'acide 4,4'-diaminodiphénylamin-2-sulfonique, la 4,4'-diaminobenzophénone, le 4,4'-diamino-diphényléther, le tétrachlorhydrate de 3,3',4,4'-tétraaminodiphényle, la 3,3',4,4'-tétraaminobenzophénone, le tétrachlorhydrate de 1,3-bis-(2,4-diaminophénoxy)-propane, le tétrachlorhydrate de 1,8-bis-(2,5-diaminophénoxy)-3,6-dioxaoctane, le 1,3-bis-(4-aminophényl-amino)-propane, le 1,3-bis-(4-aminophényl-amino)-2-propanol, 1,3-bis-[N-(4-aminophényl)-2-hydroxyéthylamino]-2-propanol, le trichlorhydrate de N,N-bis-[2-(4-aminophénoxy)-éthyl]méthylamine,
les composés hétérocycliques azotés choisis dans le groupe formé par la 2-, 3-, 4-amino-, 2-amino-3-hydroxy-, 2,6-diamino-, 2,5-diamino-, 2,3-diamino-, 2-diméthylamino-5-amino-, 3-amino-2-méthylamino-6-méthoxy-, 2,3-diamino-6-méthoxy-, 3,5-diamino-2,6-diméthoxy-, 2,4,5-triamino-, 2,6-dihydroxy-3,4-diméthylpyridine, 4,5,6-triamino-, 2-hydroxy-4,5,6-triamino-, 4-hydroxy-2,5,6-triamino-, 2,4,5,6-tétraamino-, 2-méthylamino-4,5,6-triamino-, 2,4-, 4,5-diamino-, 2-amino-4-méthoxy-6-méthyl-pyrimidine, le 2,3,4-triméthylpyrrol, le 2,4-diméthyl-3-éthylpyrrol, le 3,5-diaminopyrazole, -1,2,4-triazole, le 3-amino-, 3-amino-5-hydroxypyrazole, le 4,5-diamino-1-(2-hydroxyéthyl)-pyrazole, la 2-, 3-, 8-aminoquinoléine, la 4-aminoquinaldine, l'acide 2-, 6-aminonicotinique, la 5-aminoisoquinoléine, le 5-, 6-aminoindazole, le 5-, 7-amino-benzimidazole, -benzothiazole, la 2,5-dihydroxy-4-morpholinoaniline ainsi que les dérivés indole et indoline, comme le 4-, 5-, 6-, 7-aminoindole, le 4-, 5-, 6-, 7-hydroxyindole, le 5,6-dihydroxyindole, la 5,6-dihydroxyindoline, la 4-hydroxyindoline et les hydroxypyrimidines divulguées dans le document DE-U1-299 08 573, ainsi que respectivement les sels acceptables sur le plan physiologique de ces composés, formés avec de préférence des acides inorganiques,
les composés hydroxyaromatiques choisis dans le groupe formé par la 2-, 4-, 5-méthylrésorcine, la 2,5-diméthylrésorcine, la résorcine, le 3-méthoxyphénol, la pyrocatéchine, l'hydroquinone, le pyrogallol, la phloroglucine, l'hydroxyhydroquinone, 2-, 3-, 4-méthoxy-, 3-diméthylamino-, 2-(2-hydroxyéthyl)-, 3,4-méthylène-dioxyphénol, l'acide 2,4-, 3,4-dihydroxybenzoïque, -phénylacétique, l'acide gallique, l'acide 2,4,6-trihydroxybenzoïque, la 2,4,6-trihydroxyacétophénone, la 2-, 4-chlororésorcine, le 1-naphtol, le 1,5-, 2,3-, 2,7-dihydroxynaphtalène, l'acide 6-diméthylamino-4-hydroxy-2-naphtalènesulfonique, l'acide 3,6-dihydroxy-2,7-naphtalènesulfonique,
les acides aminés choisis dans le groupe formé par l'arginine, l'histidine, la tyrosine, la phénylalanine, la DOPA (dihydroxyphénylalanine), l'ornithine, la lysine et le tryptophane,
un oligopeptide choisi parmi le glutathion ou les oligopeptides contenus dans les hydrolysats du collagène, de la kératine, de la caséine, de l'élastine, de la protéine de soja, du gluten du blé ou de la protéine d'amande,
les composés à groupe CH actif choisis dans le groupe formé par l'iodure de 1,2,3,3-tétraméthyl-3H-indolium, le p-toluènesulfonate de 1,2,3,3-tétraméthyl-3H-indolium, le méthanesulfonate de 1,2,3,3-tétraméthyl-3H-indolium, une base de Fischer(la 1,3,3-triméthyl-2-méthylèneindoline), l'iodure de 2,3-diméthyl-benzothiazolium, le p-toluènesulfonate de 2,3-diméthyl-benzothiazolium, le p-toluènesulfonate de 1,2-diméthylnaphto[1,2-d]thiazolium, le p-toluènesulfonate de 1-éthyl-2-méthylnaphto[1,2-d]thiazolium, la rhodanine, l'acide rhodanine-3-acétique, l'iodure de 1-éthyl-2-quinaldinium, l'iodure de 1,4-diméthyl-quinoléinium, l'iodure de 1-méthyl-2-quinaldinium, l'iodure de 1,4-diméthyl-quinoléinium, l'acide barbiturique, l'acide thiobarbiturique, l'acide 1,3-diméthylthiobarbiturique, l'acide 1,3-diéthylthiobarbiturique, l'acide diéthylthiobarbiturique, l'oxindole, l'acétate de 3-indoxyle, la coumaranone, la 1-méthyl-3-phényl-2-pyrazolinone, l'indane-1,3-dione, l'indane-1-one, le 1-dicyanométhylène-indane.

6. Agent selon la revendication 5, **caractérisé en ce que** le composé supplémentaire est choisi dans le groupe formé par la N-(2-hydroxyéthyl)-N-éthyl-, 2-chloro-p-phénylènediamine, la N,N-bis-(2-hydroxyéthyl)-p-phénylènediamine, le 4-aminophénol, la p-phénylènediamine, le 2-(2,5-diaminophényl)-éthanol, le 2,5-diaminotoluène, la 3,4-méthylènedioxyaniline, le 2-amino-4-(2-hydroxyéthylamino)-anisole, le 2-(2,4-diaminophénoxy)éthanol, le 3-amino-2,4-dichloro-, 2-méthyl-5-amino-, 3-méthyl-4-amino-, 2-méthyl-5-(2-hydroxyéthylamino)-, 2-méthyl-5-amino-4-chloro-, 6-méthyl-3-amino-2-chloro-, 2-aminométhyl-4-aminophénol, le 2-diéthylaminométhyl-4-aminophénol, le 2-diméthylaminométhyl-4-aminophénol, le 2,6-dichloro-4-aminophénol, le 2-hydroxyméthyl-4-aminophénol, le 3,4-méthylènedioxyphénol, l'acide 3,4-diaminobenzoïque, la 2,5-diamino, 2-méthylamino-5-amino-, 3-amino-2-méthylamino-6-méthoxy-, 2,3-diamino-6-méthoxy-, 3,5-diamino-2,6-diméthoxy-, 2,6-dihydroxy-3,4-diméthylpyridine, la 2-hydroxy-4,5,6-triamino-, 4-hydroxy-2,5,6-triamino-, 2,4,5,6-tétraamino-, 2-méthylamino-4,5,6-triamino-pyrimidine, le 3,5-diaminopyrazole, le 3-amino-5-hydroxypyrazole, le 4,5-diamino-1-(2-hydroxyéthyl)-pyrazole, le 5,6-dihydroxyindole et la 5,6-dihydroxyindoline, la β-alanine, la L-proline, la L-lysine, la DL-tyrosine, ainsi qu'à chaque fois les sels acceptables sur le plan physiologique, de préférence formés avec des acides inorganiques, de ces composés.

7. Agent selon l'une quelconque des revendications 1 à 6, **caractérisé en ce qu'**il contient un renforçateur de couleur choisi dans le groupe formé par la pipéridine, l'acide pipéridine-2-carboxylique, l'acide pipéridine-3-carboxylique, l'acide pipéridine-4-carboxylique, la pyridine, la 2-hydroxypyridine, la 3-hydroxypyridine, la 4-hydroxypyridine, l'imidazole, le 1-méthylimidazole, l'histidine, la pyrrolidine, la proline, la pyrrolidone, l'acide pyrrolidone-5-carboxylique, le pyrazole, le 1,2,4-triazole, la pipérazidine, ou leurs mélanges quelconques.

8. Agent selon l'une quelconque des revendications 1 à 7, **caractérisé en ce qu'**il contient des colorants montant directement sur les fibres, issus des groupes des nitrophénylènediamines, des nitroaminophénols, des anthraquinones, ou des indophénols, de préférence en une quantité de 0,01 à 20% en poids, par rapport à la teinture totale.

9. Agent selon l'une quelconque des revendications 1 à 8, **caractérisé en ce que** des sels d'ammonium ou des sels métalliques sont ajoutés, choisis dans le groupe formé par les formiates, les carbonates, les halogénures, les sulfates, les butyrates, les valériates, les caproates, les acétates, les lactates, les glycolates, les tartrates, les citrates, les gluconates, les propionates, les phosphates et les phosphonates de métaux alcalins comme le potassium, le sodium ou le lithium, de métaux alcalino-terreux comme le magnésium, le calcium, le strontium ou le baryum, ou d'aluminium, de manganèse, de fer, de cobalt, de cuivre ou de zinc.

10. Agent selon l'une quelconque des revendications 1 à 9, **caractérisé en ce qu'**il contient un agent d'oxydation, en particulier H₂O₂, en une quantité de 0,01 à 6% en poids, par rapport à la solution d'application.

11. Agent selon l'une quelconque des revendications 1 à 10, **caractérisé en ce qu'**il contient des tensioactifs anioniques, zwitterioniques, ou non ioniques.

12. Utilisation de dérivés quinoxaline de formule la ou Ib ou Ic, 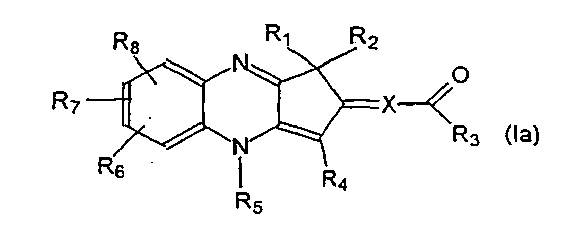 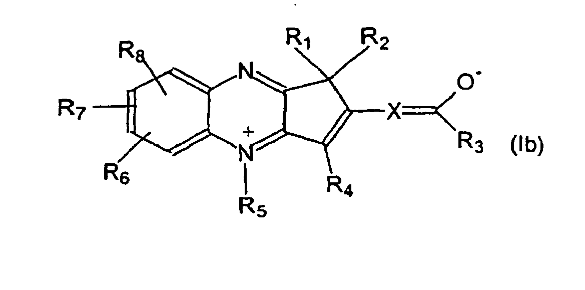 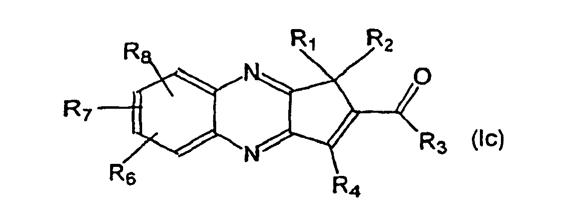 dans laquelle
R¹, R², R³, R⁴ représentent un atome d'hydrogène ou un groupe alkyle en C₁ à C₄,
R⁵ représente un atome d'hydrogène, un groupe alkyle en C₁ à C₆, ou un groupe aralkyle, aryle, alcényle en C₁ à C₄, hydroxyalkyle, carboxyalkyle,
R⁶, R⁷, R⁸ représentent un atome d'hydrogène, un atome d'halogène, un groupe alkyle en C₁ à C₄, alcoxy en C₁ à C₄, nitro, amino, alkylamino, hydroxy, carboxy, sulfo, thiol, ou alkylthio en C₁ à C₄, chacun des deux radicaux pouvant former également conjointement un cycle,
-X= où =X- représente -CH =, =CH-,
en tant que composant colorant dans des teintures capillaires par oxydation.

13. Procédé pour teindre les fibres kératiniques, en particulier les cheveux humains, dans lequel une teinture, contenant
A) au moins un dérivé quinoxaline de formule Ia ou Ib ou Ic 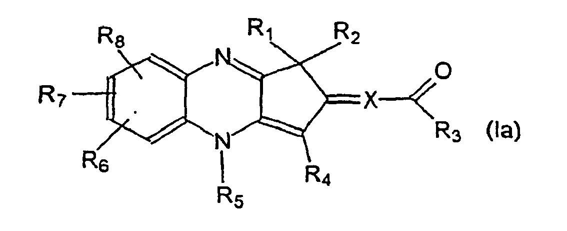 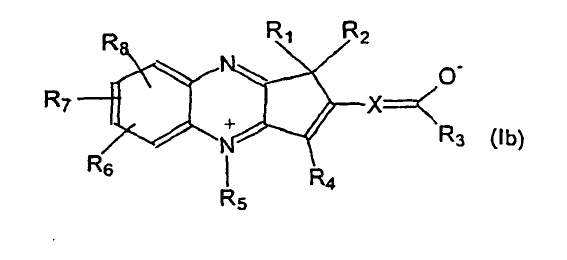 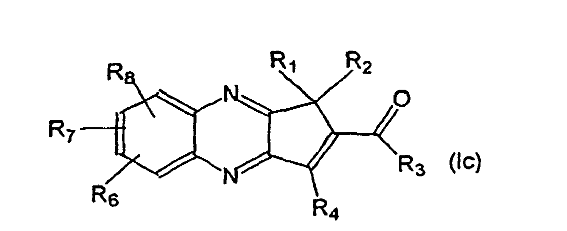 dans laquelle
R¹, R², R³, R⁴ représentent indépendamment l'un de l'autre, un atome d'hydrogène ou un groupe alkyle en C₁ à C₄,
R⁵ représente un atome d'hydrogène, un groupe alkyle en C₁ à C₆, ou un groupe aralkyle, aryle, alcényle en C₁ à C₄, hydroxyalkyle, carboxyalkyle,
R⁶, R⁷, R⁸ représentent indépendamment l'un de l'autre, un atome d'hydrogène, un atome d'halogène, un groupe alkyle en C₁ à C₄, alcoxy en C₁ à C₄, nitro, amino, alkylamino, hydroxy, carboxy, sulfo, thiol, ou alkylthio en C₁ à C₄, chacun des deux radicaux pouvant former également conjointement un cycle,
-X= où =X- représente -CH =, =CH-,
et
B) au moins un composé ayant un groupe amino primaire ou secondaire ou groupe hydroxy, choisi parmi les amines aromatiques primaires ou secondaires, les composés hétérocycliques azotés, et les composés hydroxyaromatiques, les acides aminés, les oligopeptides construits à partir de 2 à 9 acides aminés, et/ou au moins un composé à groupe CH actif,
ainsi que les ingrédients cosmétiques habituels, est appliquée sur les fibres kératiniques, et laissée quelques temps, habituellement environ 30 minutes sur la fibre, puis éliminée à nouveau par rinçage ou par lavage avec un shampoing.
